(19) Europäisches Patentamt
European Patent Office
Office européen des brevets

(11) Veröffentlichungsnummer: **0 310 555 B1**

(12) # EUROPÄISCHE PATENTSCHRIFT

(45) Veröffentlichungstag der Patentschrift: **10.02.93**

(51) Int. Cl.5: **A01N 25/32**, C07D 249/10

(21) Anmeldenummer: **88810651.5**

(22) Anmeldetag: **22.09.88**

Die Akte enthält technische Angaben, die nach dem Eingang der Anmeldung eingereicht wurden und die nicht in dieser Patentschrift enthalten sind.

(54) **1,5-Diphenyl-1,2,4,-triazol-3-carbonsäurederivate zum Schutz von Kulturpflanzen vor der phytotoxischen Wirkung von Herbiziden.**

(30) Priorität: **02.10.87 CH 3858/87**

(43) Veröffentlichungstag der Anmeldung:
**05.04.89 Patentblatt 89/14**

(45) Bekanntmachung des Hinweises auf die Patenterteilung:
**10.02.93 Patentblatt 93/06**

(84) Benannte Vertragsstaaten:
**AT BE CH DE ES FR GB GR IT LI LU NL SE**

(56) Entgegenhaltungen:
**EP-A- 0 220 956**
**DE-A- 1 123 331**
**DE-A- 2 724 819**
**DE-A- 3 525 205**

**Chemical Abstracts, Band 98, Nr. 13, 7, 15, 28.03.83, Columbus, Ohio, USA, M.A.E. KHALIFA et al. "Synthesis of azoloyl ketone and azoloylacetic acidderivates: reaction of 4-arylazo-2-oxazolin-5-ones with acti e methylene compounds" Seite 596, Zusammenfassung-Nr. 107 224b**

**Chemical Abstracts, Band 90, Nr. 9, 26.02.79, Colubus, Ohio, USA, M. RUCCIA et al: "Addition products of indazole and nitrilimines. Synthesis of 1-phenyl-5-(o-amino-phenyl)-1,2,4-triazoles and their annelation to 1,2,4-tri-azolo/1,5-f/phenanthridines", Seite 510, Zusammenfassung-Nr. 72 117z**

(73) Patentinhaber: **CIBA-GEIGY AG**
**Klybeckstrasse 141**
**CH-4002 Basel(CH)**

(72) Erfinder: **Böhner, Beat, Dr.**
**Hügelweg 3**
**CH-4102 Binningen(CH)**
Erfinder: **Moser, Hans, Dr.**
**Hauptstrasse 67B**
**CH-4312 Magden(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft 1,5-Diphenyl-1,2,4-triazol-3-carbonsäurederivate welche sich zum Schützen von Kulturpflanzen gegen schädigende Wirkungen herbizid wirksamer Phenoxyalkancarbonsäureester eignen. Die Erfindung betrifft ferner herbizide Mittel, welche eine Kombination von Herbizid und schützender 1,5-Diphenyl-1,2,4-triazolcarbonsäure oder deren Derivat enthält.

Beim Einsatz von Herbiziden wie z.B. Chloracetaniliden, N-Benzoyl-N-Phenylalaninen und Phenoxyalkancarbonsäure-Herbiziden wie beispielsweise Phenoxyphenoxy- und Pyridyloxyphenoxy-propionsäure-Derivaten können in Abhängigkeit von Faktoren wie beispielsweise Dosis des Herbizids und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in erheblichem Masse geschädigt werden. Insbesondere kann es zu starken Schädigungen kommen, wenn im Rahmen der Fruchtfolge nach Kulturpflanzen, die gegen die Herbizide resistent sind, andere Kulturpflanzen angebaut werden, welche keine oder nur unzureichende Resistenz gegenüber den Herbiziden aufweisen.

Es wurde gefunden, dass überraschenderweise ein Schutz von Kulturpflanzen gegen Schäden, welche durch die obenerwähnten Herbizide verursacht werden, durch Behandlung der Kulturpflanzen, von Teilen dieser Pflanzen oder von für den Anbau der Kulturpflanzen bestimmten Böden mit einem Safener aus einer Gruppe von 1,5-Diphenyl-1,2,4-triazol-3-carbonsäurederivaten erzielt werden kann. Die herbizide Wirkung gegenüber Unkräutern und Ungräsern wird durch diese Derivate nicht aufgehoben.

1,5-Diphenyl-1,2,4-triazol-3-carbonsäurederivate, welche zum Schützen von Kulturpflanzen vor schädigenden Wirkungen herbizid wirksamer Phenoxyphenoxy-, Benzoxazolyloxyphenoxy-, Benzthiazolyloxyphenoxy-, Chinoxalyloxyphenoxy- und Pyridyloxyphenoxy-propionsäure Derivate geeignet sind, entsprechen der Formel I

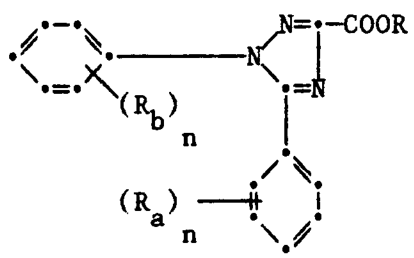

(I)

worin $R_a$ und $R_b$ unabhängig voneinander je Halogen, $C_1$-$C_5$-Alkyl, $C_1$-$C_5$-Halogenalkyl, $C_2$-$C_5$-Alkenyl, $C_2$-$C_5$-Alkinyl, $C_1$-$C_5$-Alkoxy, $C_1$-$C_5$-Halogenalkoxy, Nitro oder Cyano,
n Null oder eine Zahl von 1 bis 3 und
R Wasserstoff, ein pflanzenphysiologisch verträgliches Metall- oder Ammoniumkation, $C_1$-$C_8$-Alkyl oder $C_3$-$C_{12}$-Cycloalkyl welches unsubstituiert oder ein-oder mehrfach durch $C_1$-$C_4$-Alkoxy, $C_1$-$C_4$-Alkylthio, Mono- oder Di-$C_1$-$C_4$-alkylamino, Amino oder $C_1$-$C_4$-Alkoxycarbonyl substituiert ist; $C_3$-$C_6$-Alkenyl oder $C_3$-$C_{12}$-Cycloalkenyl, welches unsubstituiert oder ein-oder mehrfach durch Halogen substituiert ist,
$C_3$-$C_6$-Alkinyl; Phenyl oder Benzyl, welches unsubstituiert oder ein-oder mehrfach durch Halogen, Nitro, $C_1$-$C_4$-Alkyl, $C_1$-$C_4$-Alkoxy, $C_1$-$C_4$-Alkylthio, $C_1$-$C_4$-Haloalkyl, $C_1$-$C_4$-Haloalkoxy oder Cyano substituiert ist,
bedeuten.

Diese Verbindungen sind teilweise neu, teilweise bekannt. Neu sind die in mindestens einem der Phenylkerne halogenierten Verbindungen, also Verbindungen der Formel I mit der Massgabe, dass mindestens einer der Reste $R_a$ und $R_b$ Chlor oder Fluor ist.

Unter pflanzenphysiologisch verträglichen Metall- und Ammoniumkationen werden die Kationen von in Herbiziden üblichen Salzen verstanden, wie Alkalimetall-, Erdalkalimetall-, Eisen-, Kupfer-, Mangan-, oder Ammonium-, Alkylammonium-, Hydroxyalkyl- oder Alkoxyalkylammonium-Kationen.

Unter Alkylresten werden Reste mit der angegebenen Anzahl Kohlenstoffatomen verstanden. Diese Reste können geradkettig oder verzweigt sein. Die gebräuchlichsten Reste sind beispielsweise Methyl, Ethyl, n-Propyl, Isopropyl, n-Butyl, Isobutyl, sek.Butyl, tert.Butyl, n-Pentyl, Isopentyl, n-Hexyl und n-Octyl. Die Alkenyl- und Alkinylreste können ebenfalls geradkettig oder verzweigt sein und umfassen 3 bis 6 Kohlenstoffatome. Die gebräuchlichsten Reste sind beispielsweise Allyl, Methallyl, Butenyl, Butadienyl, Propinyl, Methylpropinyl, 1-Butinyl, 2-Butinyl. Cycloalkyl-oder Cycloalkenylreste haben vorzugsweise 3 - 12 Kohlenstoffatome, sie können auch benzanelliert sein. Typische Vertreter sind beispielsweise Cyclopropyl, Cyclopentyl, Cyclohexyl, Cyclohexenyl, Indanyl, Tetrahydronaphthalinyl, Decalinyl. Unter Halogen wird

Fluor, Chlor, Brom, Jod, insbesondere Fluor und Chlor verstanden. Halogenalkyl- und Halogenalkenyl-Reste können ein- oder mehrfach mit Halogen substituiert sein.

Die 1,5-Diphenyl-1,2,4-triazol-carbonsäure-Derivate der Formel I besitzen in hervorragendem Masse die Fähigkeit, Kulturpflanzen gegen die schädigende Wirkung von Herbiziden zu schützen, wie z.B. von den folgenden Chloracetaniliden: 2-Chlor-2′,6′ diethyl-N-(2″-n-propoxyethyl)-acetanieid ("Propalochlor"), 2-Chlor-6′-ethyl-N-(2″-methoxy-1″-methylethyl-acet-o-toluidid ("Metolachlor"), 2-Chlor-2′-6′-diethyl-N-(n-butoxymethyl)acetanilid ("Butachlor"), 2-Chlor-6′-ethyl-N-(ethoxymethyl)acet-o-toluidid ("Acetochlor"), 2-Chlor-6′-ethyl-N-(2″-propoxy-1″-methylethyl)aceto-o-toluidid, 2-Chlor-2′6′-dimethyl-N-(2″-methoxy-1″-methylethyl)-acetanilid, 2-Chlor-2′,6′-dimethyl-N-(2″-methoxyethyl)acetanilid ("Dimethachlor"), 2-Chlor-2′,6′-diethyl-N-(pyrazol-1-ylmethyl)acetanilid, 2-Chlor-6′-ethyl-N-(pyrazol-1-ylmethyl)acet-o-toluidid, 2-Chlor-6′-ethyl-N-(3″,5″-dimethyl-pyrazol-1-ylmethyl)acet-o-toluidid, 2-Chlor-6′-ethyl-N-(2″-n-butoxy-1″-methylethyl)acet-o-toluidid ("Metazolachlor") und 2-Chlor-2′-trimethylsilyl-N-(butoxymethyl)-acet-anilid.

Die 1,5-Diphenyl-1,2,4-triazol-3-carbonsäurederivate der Formel I schützen ebenfalls Kulturpflanzen vor der schädigenden Wirkung von herbiziden aktiven N-Benzoyl-N-phenyl-alanin-derivaten der Formel XII

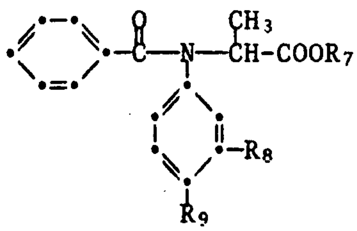 (XII)

worin

$R_7$ Wasserstoff oder $C_1$-$C_4$-Alkyl und

$R_8$ und $R_9$ unabhängig voneinander je Chlor oder Fluor bedeuten, und deren Enantiomeren, besonders von N-Benzoyl-N-(3,4-dichlorphenyl)-alanin-ethylester und N-Benzoyl-N-(3-chlor-4-fluorphenyl-)alanin-methylester.

Die 1,5-Diphenyl-1,2,4-triazol-3-carbonsäurederivate der Formel I schützen insbesondere die Kulturpflanzen vor herbizid wirksamen 2-[4-(Phenoxy-, Pyridin-2-yloxy, Benzoxazol-2-yloxy, Benzthiazol-2-yloxy- oder Chinoxalin-2-yl-oxy)-phenoxy]-propionsäure-estern der Formel XI

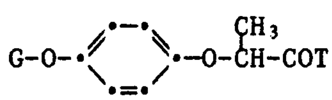 (XI),

worin G

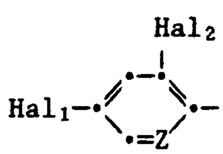 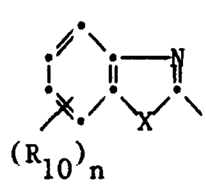 oder 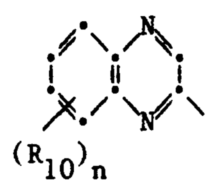 ,

$Hal_1$ Fluor, Chlor, Brom, Jod oder Trifluoromethyl, $Hal_2$ Wasserstoff, Fluor, Chlor, Brom oder Trifluoromethyl, Z für Stickstoff oder Methin (-CH=), X ein Sauerstoff oder Schwefelatom, $R_{10}$ Halogen, Trifluormethyl, Nitro, Cyan, $C_1$-$C_4$-Alkyl oder $C_1$-$C_4$-Alkoxy und n 0, 1, 2 oder 3 bedeuten.

In den Verbindungen der Formel XI bedeutet Halogen, Fluor, Chlor, Brom oder Jod, vorzugsweise Fluor, Chlor und Brom.

T steht für

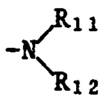 , 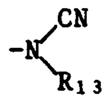 ,

$-OR_{14}$ oder $-ON=CR_{15}R_{16}$, worin

$R_{11}$, $R_{12}$: unabhängig voneinander Wasserstoff, $C_1$-$C_8$-Alkoxy, $C_1$-$C_8$-Alkyl, Phenyl oder Benzyl, $R_{11}$ und $R_{12}$ zusammen mit dem sie tragenden Stickstoffatomen einen 5-bis 6-gliedrigen gesättigten Stickstoffheterocyclus, der durch ein Sauerstoff- oder Schwefelatom unterbrochen sein kann,

$R_{13}$: $C_1$-$C_4$-Alkyl, $C_3$-$C_4$-Alkenyl, $C_3$-$C_4$-Alkinyl oder $C_2$-$C_4$-Alkoxyalkyl,

$R_{14}$: Wasserstoff oder das Aequivalent eines Alkalimetall-, Erdalkalimetall-, Kupfer- oder Eisen-Ions; einen quaternären $C_1$-$C_4$-Alkyammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, -COOR, -COSR, $-CONH_2$-, -CON($C_1$-$C_4$-alkoxy)-$C_1$-$C_4$-alkyl, -CO-N-di-$C_1$-$C_4$-alkyl, -CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest: einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR_{17}$, $-COSR_{17}$, $-CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

$R_{15}$ und $R_{16}$ unabhängig voneinander $C_1$-$C_4$-Alkyl oder zusammen eine 3-bis 6-gliedrige Alkylenkette und

$R_{17}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkenyl, $C_3$-$C_6$-Alkinyl oder $C_3$-$C_6$-Halogenalkinyl bedeuten.

In den Verbindungen der Formel I bedeutet Halogen als selbstständiger Substituent oder Teil eines anderen Substituenten, wie Halogenalkyl, Halogenalkoxy, Halogenalkenyl oder Halogenalkinyl, Fluor, Chlor, Brom oder Jod, worunter Fluor oder Chlor bevorzugt sind.

Alkyl steht je nach der Anzahl der vorhandenen Kohlenstoffatome für Methyl, Ethyl, n-Propyl, i-Propyl sowie die isomere Butyl, Pentyl, Hexyl, Heptyl oder Oktyl. Die in den Resten Alkoxy, Alkoxyalkyl, Halogenalkyl oder Halogenalkoxy enthaltenen Alkylgruppen haben die gleiche Bedeutung. Bevorzugt sind jeweils Alkylgruppen mit niedriger Anzahl von Kohlenstoffatomen.

Bevorzugte Halogenalkylreste, bzw. Halogenalkylteile in Halogenalkoxyresten sind: Fluormethyl, Difluormethyl, Trifluormethyl, Chlormethyl, Trichlormethyl, 2-Fluorethyl, 2,2,2-Trifluorethyl, 1,1,2,2-Tetrafluorethyl, Perfluorethyl, 2-Chlorethyl, 2,2,2-Trichlorethyl, 2-Bromethyl und 1,1,2,3,3,3-Hexafluorpropyl.

Cycloalkyl steht für mono-, und bi-cyclische gesättigte Kohlenwasserstoffringsysteme wie Cyclopropyl, Cyclobutyl, Cyclopentyl, Cyclohexyl, Cycloheptyl, Cyclooktyl, Cyclononyl, Bicyclo[4.3.0]nonyl, Bicyclo-[5.2.0]nonyl oder Bicyclo[2.2.2.]oktyl.

Besonders bemerkenswert ist die Schutzwirkung von Triazol-Derivaten der Formel I gegenüber solchen Herbiziden der Formel XI, in denen T für die Gruppen $-O-R_{14}$ oder $-O-N=CR_{15}R_{16}$ steht, wobei $R_{14}$ Wasserstoff, $C_1$-$C_4$-Alkyl, $C_3$-$C_4$-Alkinyl oder durch $C_1$-$C_4$-Alkoxycarbonyl oder Di-$C_1$-$C_4$-alkylamino substituiertes $C_1$-$C_4$-Alkyl und

$R_{15}$ und $R_{16}$ unabhängig voneinander $C_1$-$C_4$-Alkyl oder

$R_{15}$ und $R_{16}$ zusammen eine $C_4$-$C_7$-Alkylenkette bedeuten.

Besonders hervorzuhebende Einzelbedeutungen für T sind dabei Methoxy, Ethoxy, Propyloxy, Isopropyloxy, Butyloxy, Dimethylaminoethoxy, Propargyloxy, 1-Cyano-1-methylethoxy, Methoxycarbonylmethylthio, 1-Aethoxycarbonylethoxy, Butyloxycarbonylmethoxy, $-O-N=C(CH_3)_2$, $-O-N=C(CH_3)C_2H_5$ oder $-O-N=C(CH_2)_5$, und für G

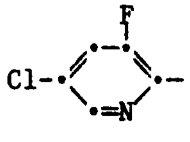 , 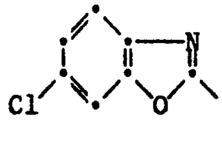 oder 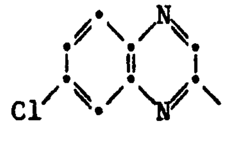 .

Bevorzugte Einzelverbindungen der Formel XI sind:

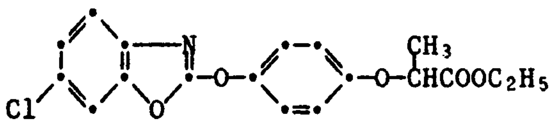

,

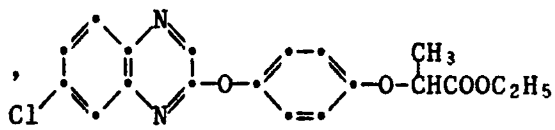

sowie die 2-Pyridyloxyphenoxy-propionsäure-Derivate der angeschlossenen Tabelle 1.

Tabelle 1

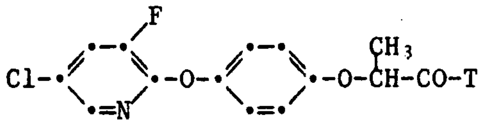 (XI)

| Nr. | T | physikalische Konstante |
|---|---|---|
| 1.1 | $-OCH_3$ | Smp. 63-64°C |
| 1.2 | $-OC_4H_9$-n | $n_D^{35}$ = 1.5275 |
| 1.3 | $-O-N{=}C(CH_3)_2$ | $n_D^{35}$ = 1.5488 |
| 1.4 | $-OC_2H_5$ | $n_D^{35}$ = 1.5358 |
| 1.5 | $-O-CH_2-CH_2-N(CH_3)_2$ | $n_D^{35}$ = 1.5334 |
| 1.6 | $-O-CH_2-C{\equiv}CH$ | $n_D^{35}$ = 1.5492 |
| 1.7 | 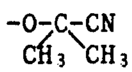 | $n_D^{35}$ = 1.5330 |
| 1.8 | $-S-CH_2-COOCH_3$ | $n_D^{35}$ = 1.5607 |
| 1.9 | 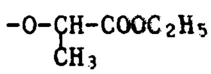 | $n_D^{35}$ = 1.5227 |
| 1.10 | $-O-CH_2-COOC_4H_9$-n | $n_D^{35}$ = 1.5223 |
| 1.11 | $-OC_3H_7$-n | $n_D^{35}$ = 1.5319 |
| 1.12 | $-OC_3H_7$-i | $n_D^{35}$ = 1.5284 |
| 1.13 | 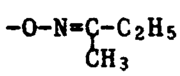 | $n_D^{35}$ = 1.5340 |
| 1.14 | 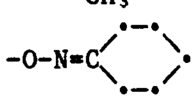 | $n_D^{35}$ = 1.5360 |
| 1.15 | $-OCH_3$ (2R) | $n_D^{35}$ = 1.5359 |
| 1.16 | -OH | Smp. 95-97°C |
| 1.17 | $-S-CH_2-COOCH_3$ (2R) | $n_D^{35}$ = 1.5623 |

Tabelle 1 (Fortsetzung)

| Nr. | T | physikalische Konstante |
|---|---|---|
| 1.18 | $-O-CH-COOC_2H_5$ (2R,S)<br>$CH_3$ | $n_D^{35}$ = 1,5223 |
| 1.19 | $-O-CH_2-C{\equiv}CH$ (2R) | Smp. 55-56°C |
| 1.20 | $-NH-OCH_3$ | Smp. 103-105°C |

Insbesondere ist die Einzelverbindung 1.6

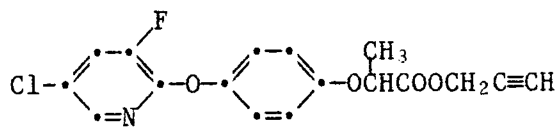

oder sein 2R Enantiomeres (Verbindung 1.19) hervorzuheben.

Wegen des optisch aktiven Kohlenstoffatoms in der Propionsäure können diese Verbindungen sowohl in den R- als auch S-Konfigurationen vorkommen. Ohne besondere Angabe sind hierin die racemischen Gemische gemeint. Bevorzugte Herbizide der Formel I sind 2R-konfiguriert.

Als Kulturpflanzen, welche durch 1,5-Diphenyl-1,2,4-triazol-3-carbonsäuren und Derivate der Formel I gegen schädigende Wirkungen der oben erwähnten Herbiziden geschützt werden können, kommen insbesondere diejenigen in Betracht, die auf dem Nahrungs- oder Textilsektor von Bedeutung sind, beispielsweise Zuckerrohr und insbesondere Kulturhirse, Mais, Reis und andere Getreidearten (Weizen, Roggen, Gerste, Hafer).

Die 1,5-Diphenyl-1,2,4-triazol-3-carbonsäurederivate der Formel I sind z.T. bekannte, z.T. neue Verbindungen. Neu und als Antidots gegen die obgenannten Herbizide besonders wirksam sind diejenigen, in denen keiner der Substituenten $R_a$ oder $R_b$ Nitro und mindestens einer Chlor oder Fluor bedeuten, insbesondere diejenigen, welche der Formel Ia

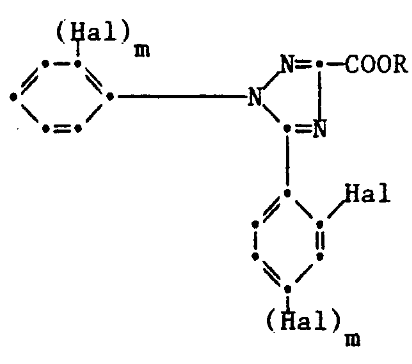

(Ia)

worin Hal ein Halogenatom, m Null oder 1 bedeuten und R die unter der Formel I gegebene Bedeutung hat, entsprechen. Als sehr gute Antidots haben sich ferner die Verbindungen der Formel Ib

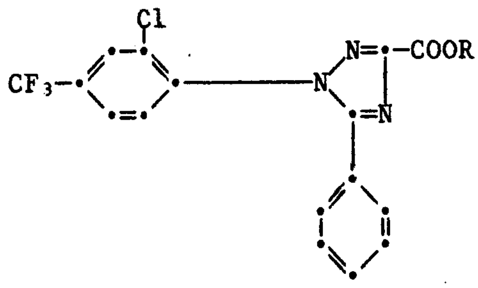

(Ib)

erwiesen, worin R die unter der Formel I gegebene Bedeutung hat, insbesondere aber Methyl oder Ethyl bedeutet.

Die 1,5-Diphenyl-1,2,4-triazol-3-carbonsäuren und Derivate der Formel I

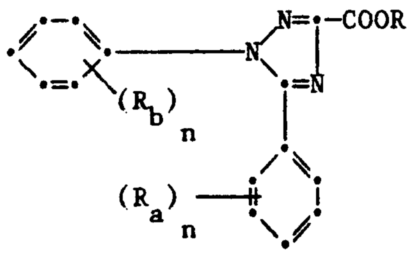

(I)

können nach verschiedenen, an sich bekannten Synthesewegen hergestellt werden.

Ihre Herstellung ist z.B. in der folgenden Literatur beschrieben, worin 1,5-Diphenyl-1,2,4-triazol-3-carbonsäurederivate als Zwischen- oder Endprodukte erwähnt sind:

DE-PS 1 123 331,
US-PS 4 492 597,
EP-A 220 956,
J. Chem. Soc. 1962 575-583,
J. Chem. Soc. 1985 103-105,
J. Chem. Soc. 79 1955-56 (1957),
Chem. and Ind. 1960 1086,
Synthesis 1986 772,
J. Am. Chem. Soc. 79 (1957) 1955,
J. Chem. Soc. 87 (1905) 1859,
Chem. Ber. 50 (1917) 1482.

Wir haben die 1,5-Diphenyl-1,2,4-triazol-3-carbonsäurederivate gemäss den in den folgenden Reaktionsschemen dargestellten Synthesewegen hergestellt.

Nach einem ersten, erfindungsgemässen Verfahren wird das Phenylhydrazin der Formel VII mit einem Thiooxamidsäurealkylester kondensiert, wobei Schwefelwasserstoff abgespalten wird. Das entstandene Kondensationsprodukt der Formel IX wird dann durch Erhitzen mit einem Orthobenzoesäuretrialkylester der Formel X, z.B. dem Triethylat, zum 1,5-Diphenyl-1,2,4-triazol-3-carbonsäurederivat der Formel I ringgeschlossen.

Schema 1:

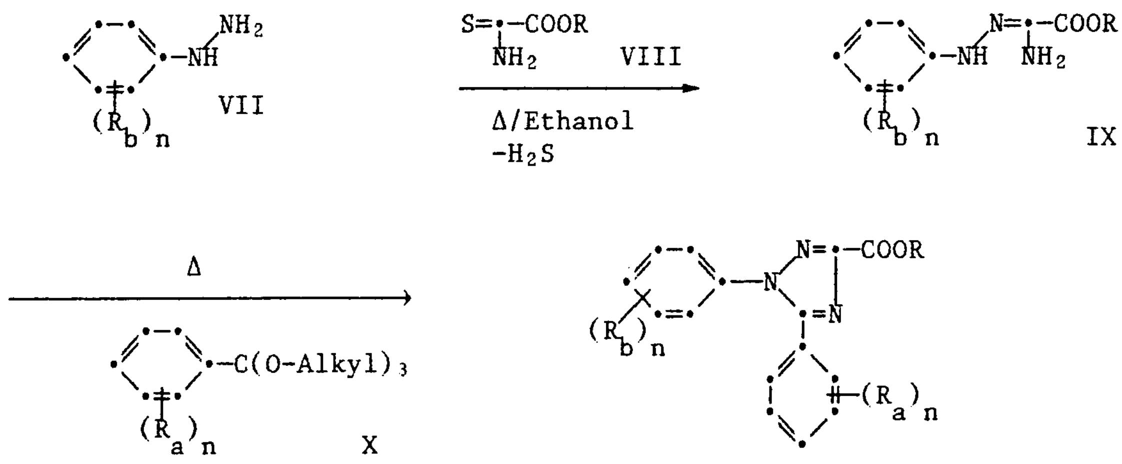


Nach einem zweiten Verfahren analog zu J. Chem. Soc. 87 (1905) 1859, reagiert man ein Anilin mit Natriumnitrit und Salzsäure und gibt zur entstandenen Diazoverbindung einen 2-Chloracetessigsäurealkylester. Das Kondensationsprodukt wird mit Ammoniak behandelt und mit einem Benzoylchlorid zum 1,5-Diphenyl-1,2,4-triazol-3-carbonsäurederivat ringgeschlossen.

Schema 2:

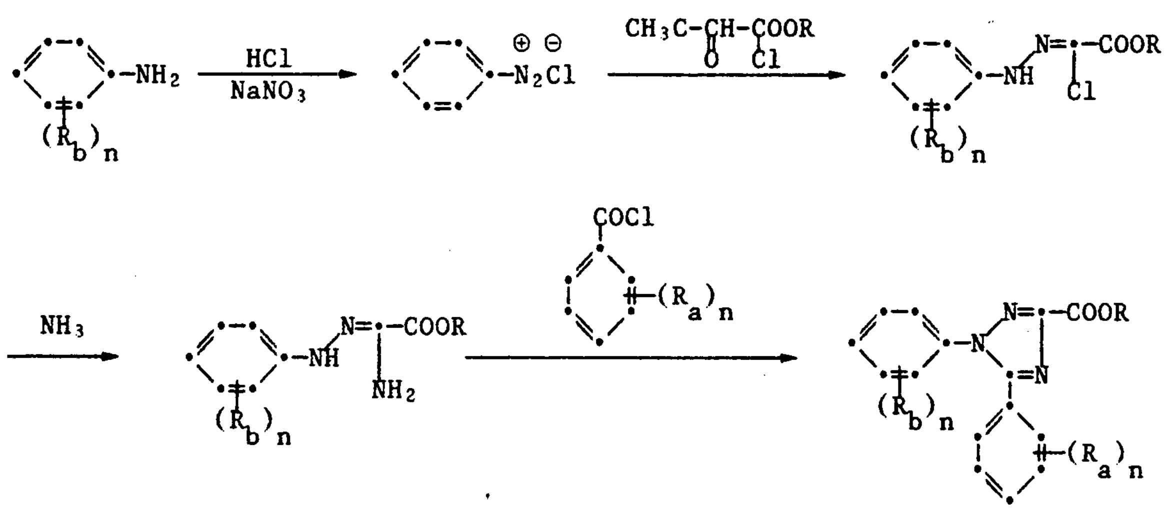


Nach einem dritten Verfahren analog zu DE-PS 1 123 331 wird zuerst ein Anilin mittels Natriumnitrit und Salzsäure diazotiert und dann wird ein Benzoylaminomalonsäuredialkylester zugefügt. Das Kondensationsprodukt wird anschliessend unter wasserabspaltenden Bedingungen hier, z.B. mit Natriummethylat in Methanol, zum 1,5-Diphenyl-1,2,4-triazol-3-carbonsäurederivat ringgeschlossen.

Schema 3:

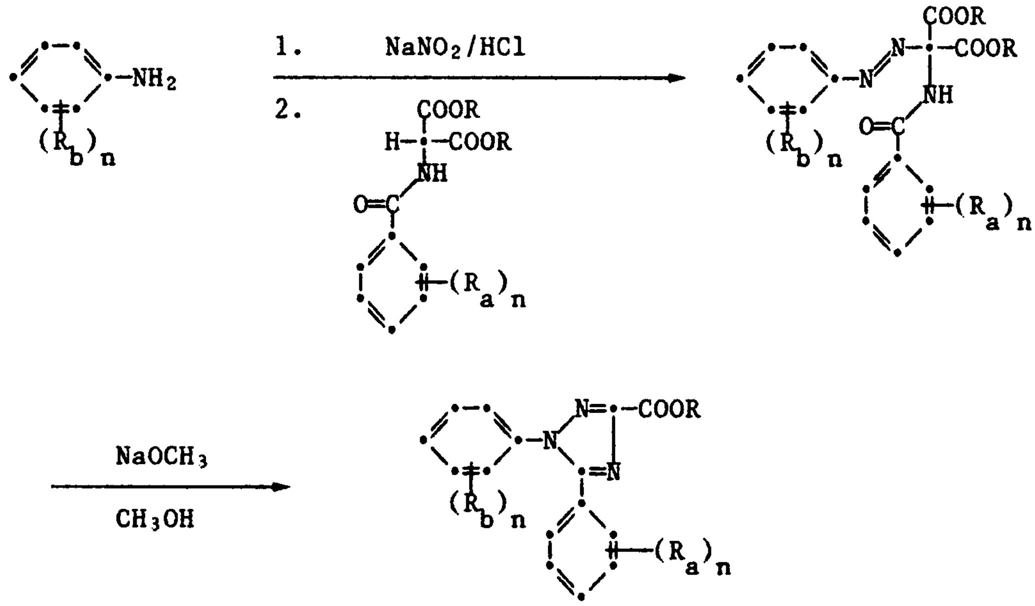


Ein allgemeines, teilweise in J. Am. Chem. Soc. 79 (1957) 1955 beschriebenes Verfahren zur Herstellung der 1,5-Diphenyl-1,2,4-triazol-3-carbonsäurederivate der Formel I kann durch das folgende Schema 4 wiedergegeben werden:

Schema 4:

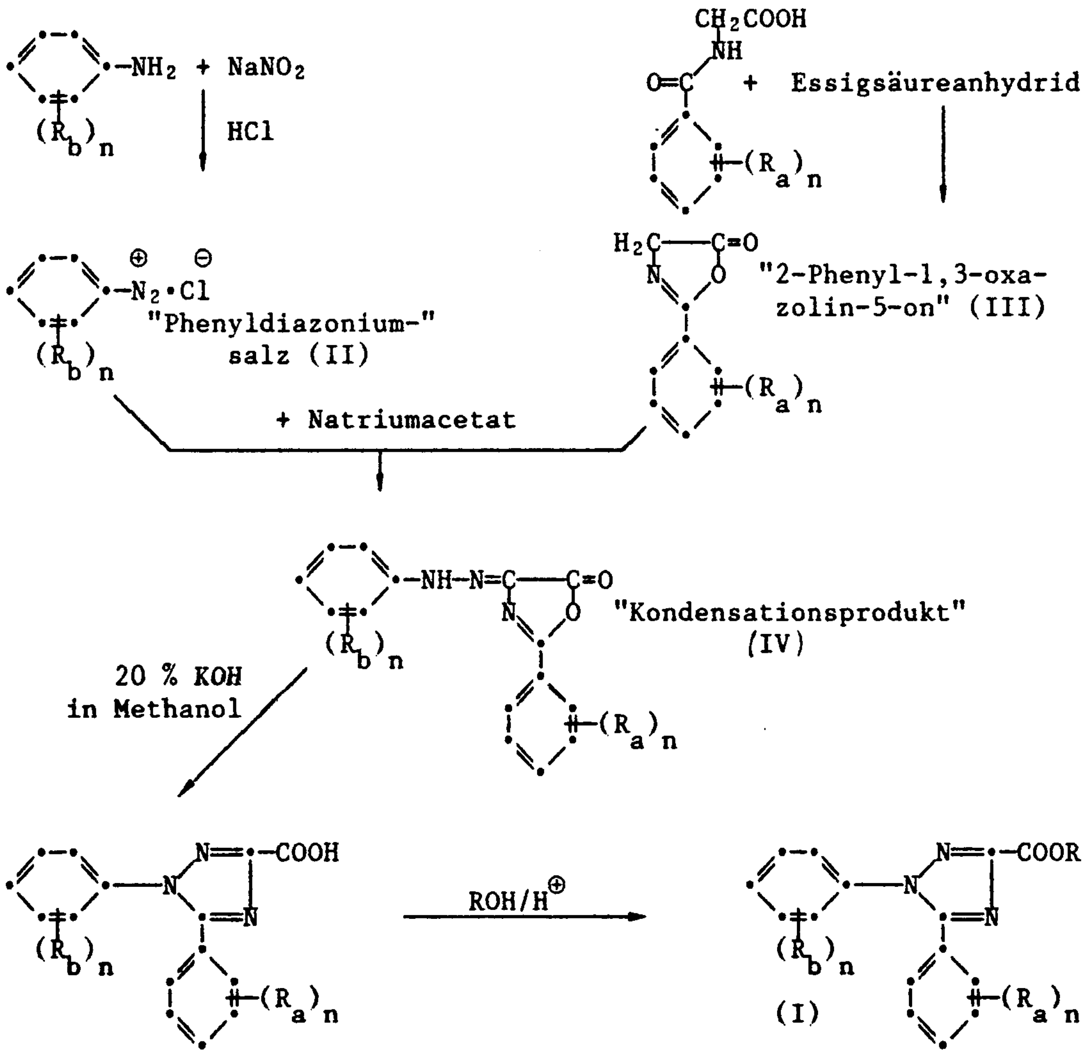


1,5-Diphenyl-1,2,4-triazol-3-carbonsäure (Ic)

Das erfindungsgemässe Verfahren zur Herstellung der 1,5-Diphenyl-1,2,4-triazol-3-carbonsäurederivate der Formel I ist dadurch gekennzeichnet, dass man ein Phenyldiazoniumsalz der Formel II

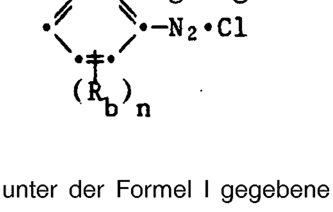

(II)

worin n und $R_b$ die unter der Formel I gegebene Bedeutung haben, mit einem 2-Phenyl-1,3-oxazolin-5-on der Formel III

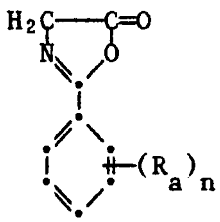

(III)

worin n und $R_a$ die unter der Formel I gegebene Bedeutung haben, in Gegenwart von Natriumacetat kondensiert und das erhaltene Kondensationsprodukt der Formel IV

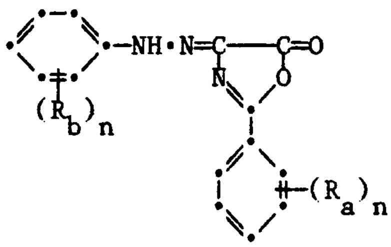

(IV)

worin n, $R_a$ und $R_b$ die unter Formel I gegebene Bedeutung haben, durch Erwärmen ringschliesst, und die entstandene 1,5-Diphenyl-1,2,4-triazol-3-carbonsäure der Formel Ic

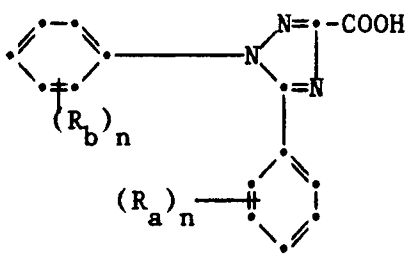

(Ic)

gewünschtenfalls mit einem Alkohol der Formel V

HO-R (V)

worin R die unter der Formel I gegebene Bedeutung hat, verestert.

Diese Reaktionsschritte und Variationen davon sind in der Literatur, z.B. in der obgenannten Referenz, beschrieben. Die Ausgangsprodukte sind bekannt.

Als Lösungsmittel kommen für die Kondensation entsprechende Alkohole, wie Methanol oder Ethanol; Aceton und höhersiedende Ketone, wie Methylethylketon; aber auch höhersiedende Ether wie Dipropylether, Dioxan oder Tetrahydrofuran; ferner aromatische Kohlenwasserstoffe, wie Benzol, Toluol oder Xylol in Betracht.

Die Reaktionstemperaturen liegen zwischen 0 °C und 200 °C. Vorzugsweise wird jedoch zwischen 0 °C und dem Siedepunkt des Reaktionsgemisches gearbeitet.

In den nachfolgenden Beispielen ist die Herstellung erfindungsgemässer 1,5-Diphenyl-1,2,4-triazol-3-carbonsäurederivate beschrieben. Temperaturen sind in Grad Celsius angegeben.

Beispiele für weitere erfindungsgemäss zu verwendende Verbindungen mit Schutzwirkung gegen Herbizide sind in der darauffolgenden Tabelle 2 aufgeführt.

Beispiel 1

Herstellung von 1-(2-Chlor-4-trifluormethylphenyl)-5-phenyl-1,2,4-triazol-3-carbonsäure-ethylester.

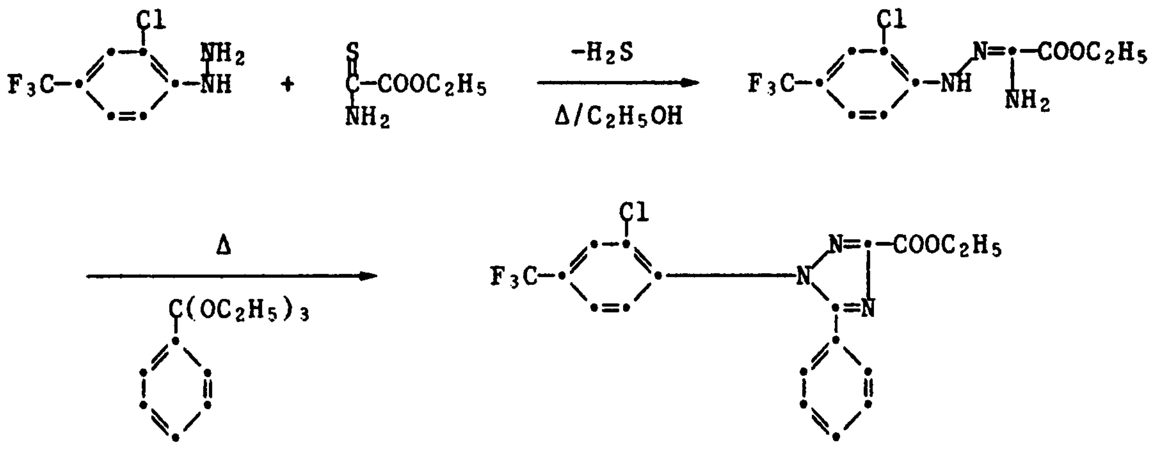


Ein Gemisch von 13,3 g (0,1 Mol) Thiooxamidsäure-ethylester und 21,0 g (0,1 Mol) 2-Chlor-4-trifluormethyl-phenylhydrazin wird in 400 ml absolutem Ethanol für zwei Stunden zum Rückfluss erhitzt. Der sich dabei abspaltende Schwefelwasserstoff wird zur Neutralisierung durch Natriumhypochlorit-Lösung geleitet. Die klare, rote Reaktionslösung wird eingedampft und der Rückstand mit Toluol über eine kurze Kieselgelsäule gereinigt. Nach dem Eindampfen der Hauptfraktion des Eluates erhält man 21,3 g (68,8 % der Theorie) N-(2-Chlor-4-trifluormethylphenyl)-N′-amino-N′-ethoxycarbonyl-hydrazon mit einem Schmelzpunkt von 97-98 °C, welches zusammen mit 15,6 ml (0,069 mol) Orthobenzoesäure-triethylester aufgeheizt wird. Bei einer Badtemperatur von 125 °C beginnt Aethanol abzudestillieren. Nachdem mittels Dünnschichtchromatographie das Ende der Reaktion festgestellt worden ist, kühlt man ab. Der Rückstand wird mit Hexan verrieben und das auskristallisierte, hellbeige Produkt abfiltriert. Man erhält 22,4 g (82,7 %) der Theorie 1-(2-Chlor-4-trifluormethylphenyl)-5-phenyl-1,2,4-triazol-3-carbonsäure-ethylester mit einem Schmelzpunkt von 107-108 °C.

Beispiel 2

Herstellung von 1-(3-Chlorphenyl)-5-(2-fluorphenyl)-1,2,4-triazol-3-carbonsäure-ethylester.

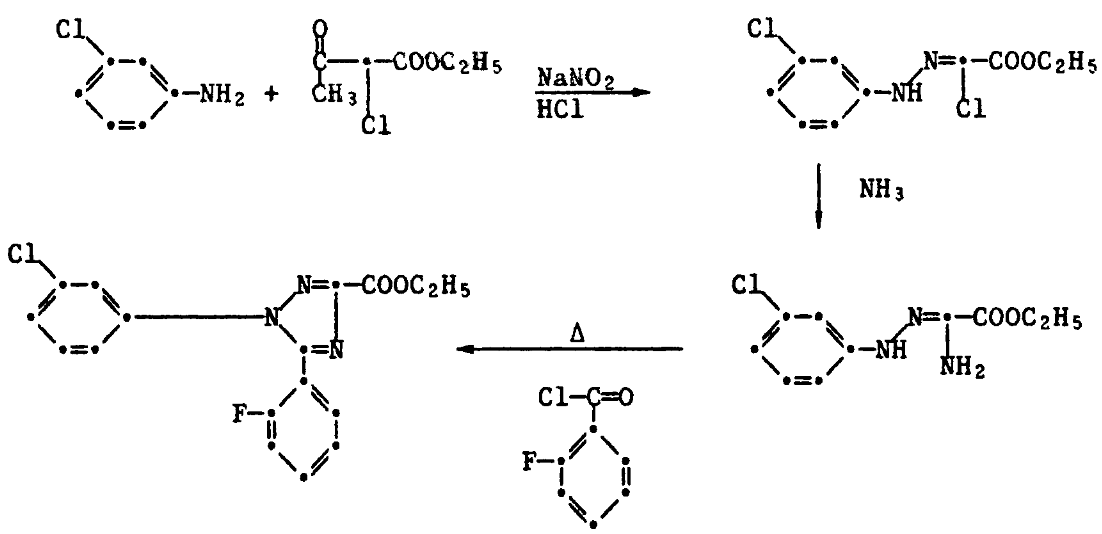


Zu einer Suspension von 25,5 g (0,2 Mol) 3-Chloranilin in 80 ml Wasser und 65 ml konzentrierter Salzsäure lässt man bei 0 °C eine Lösung von 14,0 g (0,202 Mol) Natriumnitrit in 80 ml Wasser zutropfen. Nach 15 Min. Ausrühren lässt man die schaumige, braune Lösung in ein Gemisch von 33,1 g (0,201 mol) 2-Chloracetessigsäureethylester, 90 g Natriumacetat in 160 ml Wasser und 200 ml Ethanol einlaufen.

Anschliessend lässt man bei 10 °C während drei Stunden ausrühren. Nach Zugabe von 1 l Wasser scheidet sich ein rotes Oel ab, welches mit Essigester extrahiert wird. Nach dem Trocknen mit Magnesiumsulfat und Einengen des Extrakts erhält man in quantitativer Ausbeute ein orangerotes, kristallines Material, das in 320 ml Tetrahydrofuran gelöst wird. Nach dem Zutropfen von 35 g (0,625 Mol) 25 %igen wässrigem Ammoniak bei 15-20 °C wird für zwei Stunden gerührt. Das Ende der Reaktion wird durch Dünnschichtchromatographie festgestellt. Dann gibt man 1 l Wasser zu und extrahiert das ausgefallene Oel mit Essigester. Nach dem Trocknen und Eindampfen des Extraktes erhält man 40,7 g (84,4 % der Theorie) eines Zwischenproduktes. 4,8 g (0,02 Mol) davon werden in 30 ml Toluol gelöst und mit 2,5 ml (0,021 Mol) 2-Fluorbenzoylchlorid versetzt. Die klare, braune Reaktionslösung wird erwärmt, bis sich Wasser und Salzsäure abscheiden und das Lösungsmittel abdestilliert ist. Der Rückstand wird über eine kurze Kieselgelsäule vorgereinigt und dann aus Ether/Hexan umkristallisiert. Man erhält 3,6 g (52,2 % Theorie) 1-(3-Chlorphenyl)-5-(2-fluorphenyl)-1,2,4-triazol-3-carbonsäure-ethylester als weissbeige Kristalle, welche bei 77-78 °C schmelzen.

Beispiel 3

Herstellung von 1-(4-Chlorphenyl)-5-phenyl-1,2,4-triazol-3-carbonsäuremethylester.

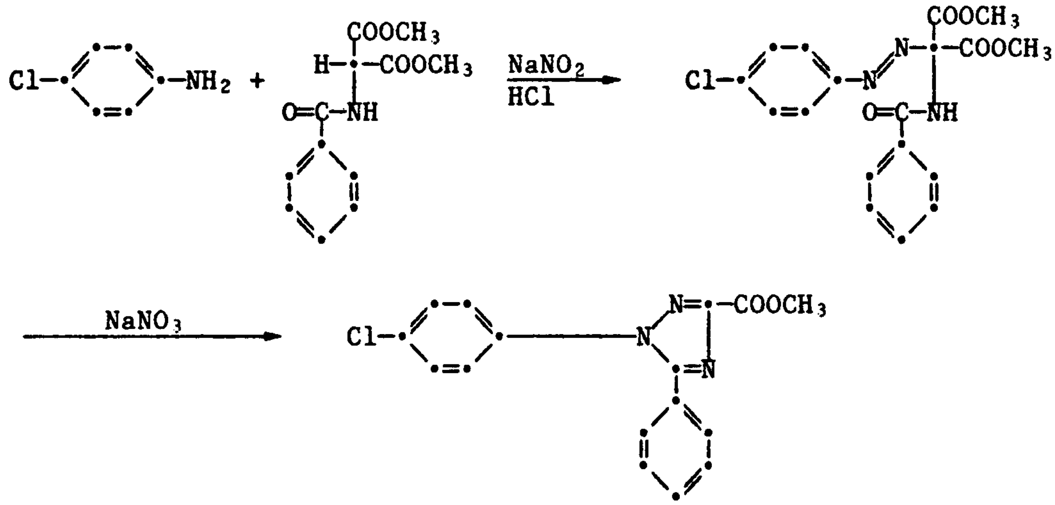


Man löst 6,4 g (0,05 Mol) 4-Chloranilin in einem Gemisch von 50 ml Eisessig und 12,5 ml konzentrierter Salzsäure, kühlt auf 0 °C ab, und versetzt tropfenweise mit einer Lösung von 3,5 g (0,0507 Mol) Natriumnitrit in 6,5 ml Wasser. Das Reaktionsgemisch wird dann auf -10 °C gekühlt und tropfenweise mit einer Lösung von 11,7 g (0,0466 Mol) Benzoylaminomalonsäure-dimethylester in 120 ml Aceton versetzt. Dann wird eine Lösung von 70,2 g (0,509 Mol) Kaliumcarbonat in 70 ml Wasser zugetropft und noch eine halbe Stunde bei 0-5 °C nachgerührt. Danach wird Essigester zum Reaktionsgemisch gegeben. Die organische Phase wird abgetrennt, mit Wasser gewaschen, über Natriumsulfat getrocknet, filtriert und eingedampft. Dabei entsteht in quantitativer Ausbeute ein Zwischenprodukt als rotes, klares Oel, von welchem 9,75 g (0,025 Mol) in 50 ml Methanol gelöst und mit 0,7 g (0,004 Mol) 30 %igem Natriummethylat in Methanol versetzt werden. Man lässt die Reaktionslösung über Nacht bei Raumtemperatur stehen, wobei das Produkt auskristallisiert. Nach dem Abfiltrieren und Waschen mit wenig Methanol erhält man 3,4 g (43,6 % der Theorie) 1-(4-Chlorphenyl)-5-phenyl-1,2,4-triazol-3-carbonsäure-methylester mit einem Schmelzpunkt von 136-137 °C.

Analog zu diesen Beispielen werden folgende Verbindungen hergestellt:

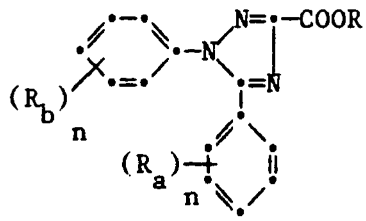

Tabelle 2

| No. | $(R_b)_n$ | $(R_a)_n$ | R | phys. Daten |
|---|---|---|---|---|
| 2.001 | 3-Cl | - | $CH_3$ | Smp 127-129° |
| 2.002 | 2-Cl,4-$CF_3$ | - | $C_2H_5$ | Smp 107-108° |
| 2.003 | - | - | $C_2H_5$ | Smp 163-163° |
| 2.004 | 4-Cl | - | $C_2H_5$ | Smp 130-131° |
| 2.005 | 2-Cl, 4-Cl | - | $C_2H_5$ | Smp 103-104° |
| 2.006 | 2-F,4-Cl,5-$OCH(CH_3)_2$ | - | $C_2H_5$ | Smp 131-132° |
| 2.007 | 3-Cl | 4-Cl | $CH_3$ | Smp 113-114° |
| 2.008 | 3-$CH_3$ | 2-Cl | $CH_3$ | Oel |
| 2.009 | 3-$CH_3$ | 2-F | $CH_3$ | Smp 79-80° |
| 2.010 | 2-Cl | 2-Cl | $CH_3$ | Smp 146-147° |
| 2.011 | 4-Cl | - | $CH_3$ | Smp 136-137° |
| 2.012 | - | 2-Cl | $C_2H_5$ | Smp 132-134° |
| 2.013 | 2-Cl | - | $C_2H_5$ | Smp 121-122° |
| 2.014 | 2-$NO_2$ | - | $C_2H_5$ | Smp 148-149° |
| 2.015 | 2-$CH_3$ | - | $C_2H_5$ | Smp 114-115° |
| 2.016 | 3-Cl | 2-F | $C_2H_5$ | Smp 77-78° |
| 2.017 | - | 2-F | $C_2H_5$ | Smp 93-94° |
| 2.018 | - | 2-Cl | $C_2H_5$ | $n_D^{22}$ 1.5088 |
| 2.019 | 2-Cl,5-$CF_3$ | - | $C_2H_5$ | Smp 157-158° |
| 2.020 | 2-$CH_3$,4-Cl | - | $C_2H_5$ | Smp 109-110 |
| 2.021 | 2-F,5-$CF_3$ | - | $C_2H_5$ | Smp 151-152° |
| 2.022 | 2-Cl | - | $CH_3$ | Smp 104-106° |
| 2.023 | - | - | $CH_3$ | Smp 160-162° |
| 2.024 | 2-Cl,4-$CF_3$ | - | $CH_3$ | |
| 2.025 | 2-Cl,4-$CF_3$ | - | $C_3H_7$-n | |
| 2.026 | 2-Cl,4-$CF_3$ | - | $CH_2CH{=}CH_2$ | |
| 2.027 | 2-Cl,4-$CF_3$ | - | $CH_2C{\equiv}CH$ | |

| No. | $(R_b)_n$ | $(R_a)_n$ | R | phys. Daten |
|---|---|---|---|---|
| 2.028 | 2-Cl | 2-Cl | $C_2H_5$ | |
| 2.029 | 2-Cl | 2-Cl | $CH(CH_3)_2$ | |
| 2.030 | 2-Cl | 2-Cl | $C_4H_9$-n | |
| 2.031 | 2-Cl | 2-Cl | $CH_2C{\equiv}CH$ | |
| 2.032 | 3-Cl | 2-F | $CH_3$ | |
| 2.033 | 3-Cl | 2-F | $C_5H_{11}$-n | |
| 2.034 | 2-Cl | 2-F | $CH_3$ | Smp 84-87° |
| 2.035 | - | 2-F | $C_3H_7$-n | |
| 2.036 | - | 2-F | $CH_2CH{=}CH_2$ | |
| 2.037 | - | 2- F | $CH_2COOCH_3$ | |
| 2.038 | - | 2-F | $C_2H_4OCH_3$ | |
| 2.039 | - | 2-Cl | $CH_3$ | |
| 2.040 | - | 2-Cl | $C_3H_7$-n | |
| 2.041 | - | 2-Cl | Benzyl | |
| 2.042 | - | 2-Cl | 4-Methoxyphenyl | |
| 2.043 | 2-Cl | - | $C_4H_9$-n | |
| 2.044 | 2-Cl | - | $C_2H_4N(C_2H_5)_2$ | |
| 2.045 | 2-Cl | - | $C_3H_6Cl$ | |
| 2.046 | 2-Cl | - | $C_2H_4SCH_3$ | |
| 2.047 | 2-Cl | - | $CH_2COOC_2H_5$ | |
| 2.048 | 2-Cl | - | 2-Chlorbenzyl | |
| 2.049 | 2-$CH_3$ | - | $CH_3$ | Smp 138° |
| 2.050 | 3-$CH_3$ | - | $CH_3$ | Smp 159° |
| 2.051 | 4-$CH_3$ | - | $CH_3$ | Smp 152° |
| 2.052 | 4-Br | - | $CH_3$ | Smp 168° |
| 2.053 | 4-$OCH_3$ | - | $CH_3$ | Smp 153° |
| 2.054 | - | - | H | Smp 177-178° |
| 2.055 | - | - | $CH_3$ | Smp 158-159° |
| 2.056 | 2-$CH_3$ | - | H | Smp 171-172 |
| 2.057 | 3-$CH_3$ | - | H | Smp 183-184° |
| 2.058 | 4-$CH_3$ | - | H | Smp 177-178° |
| 2.059 | - | 2-F | $CH_3$ | |

| No. | ($R_a$) | ($R_b$) | R | phys. Daten |
|---|---|---|---|---|
| 2.059 | 4-$OCH_3$ | - | H | Smp 176-177° |
| 2.060 | 4-Br | - | H | Smp 179-180° |
| 2.061 | - | 4-Br | $CH_3$ | Smp 157-159° |
| 2.062 | - | - | $C_2H_5$ | Smp 176° |
| 2.063 | 3-$CH_3$ | - | $C_2H_5$ | |
| 2.064 | - | 4-$NO_2$ | $CH_3$ | Smp 116-118° |
| 2.065 | 4-$NO_2$ | - | $CH_3$ | Smp 179-181° |

Die erfindungsgemässen 1,5-Diphenyl-1,2,4-triazol-3-carbonsäuren und Derivate der Formel I werden als Safener in Mischung mit Herbiziden, Chloracetaniliden, N-Benzoyl-N-phenyl-anilinderivaten oder 2-[4-(Phenoxy, Pyridin-2-yloxy-, 4-Benzoxazol-2-yloxy, 4-Benzthiazol-2-yloxy oder 4-Chinoxalin-2-yloxy)-phenoxy]-propionsäureester-Herbiziden zur Bekämpfung von Unkräutern in Nutzpflanzenkulturen verwendet.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehend genannten in Betracht. Als Anbauflächen gelten die bereits mit den Kulturpflanzen bewachsenen oder mit dem Saatgut dieser Kulturpflanzen beschickten Bodenareale, wie auch die zur Bebauung mit diesen Kulturpflanzen bestimmten Böden.

Ein Gegenmittel oder Antidot der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidot kan daher grundsätzlich unabhängig vom Zeitpunkt der Applikation der phytotoxischen Chemikalie erfolgen. Die Behandlung der Pflanze kann jedoch auch durch gleichzeitige Applikation von phytotoxischer Chemikalie und Gegenmittel (Tankmischung) erfolgen. Die preemergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die zu applizierende Aufwandmenge Antidot im Verhältnis zum Herbizid richtet sich weitgehend nach der Anwendungsart. Bei einer Feldbehandlung, welche entweder unter Verwendung einer Tankmischung mit einer Kombination von Antidot und Herbizid oder durch getrennte Applikation von Antidot und Herbizid erfolgt, liegt in der Regel ein Verhältnis von Antidot zu Herbizid von 1:100 bis 10:1, bevorzugt 1:20 bis 1:1, und insbesondere 1:1, vor. Dagegen werden bei der Samenbeizung weit geringere Mengen Antidot im Verhältnis zur Aufwandmenge an Herbizid pro Hektar Anbaufläche benötigt.

In der Regel werden bei der Feldbehandlung 0,001 bis 1,0 kg Antidot/ha, vorzugsweise 0,01 bis 0,1 kg Antidot/ha, appliziert,

Bei der Samenbeizung werden im allgemeinen 0,01 bis 10 g Antidot/kg Samen, vorzugsweise 0,05 bis 2 g Antidot/kg Samen, appliziert. Wird das Antidot in flüssiger Form kurz vor der Aussaat unter Samenquellung appliziert, so werden zweckmässigerweise Antidot-Lösungen verwendet, welche den Wirkstoff in einer Konzentration von 1 bis 10 000, vorzugsweise von 100 bis 1 000 ppm, enthalten.

Zur Applikation werden die Verbindungen der Formel I oder Kombinationen von Verbindungen der Formel I mit den zu antagonisierenden Herbiziden zweckmässigerweise zusammen mit den in der Formulierungstechnik üblichen Hilfsmittel eingesetzt und werden daher z.B. zu Emulsionkonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen, Bestreichen oder Giessen werden gleich wie die Art der zu verwendenden Mittel den angestrebten Zielen und den gegebenen Verhältnissen entsprechend gewählt.

Die Formulierungen, d.h. die den Wirkstoff der Formel I oder eine Kombination von Wirkstoff der Formel I mit antagonisierendem Herbizid und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmiteln, wie z.B. mit Lösungsmitteln, festen

Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoff wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann ein Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch dem zu antagonisierenden Herbizid nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na-oder K-Salze oder Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppe und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure- Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanolerwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood New Jersey, 1981.
Stache, H., "Tensid-Taschenbuch",
Carl Hanser Verlag, München/Wien, 1981.

Die agrochemischen Zubereitungen enthalten in der Regel 0,1 bis 99 Gewichtsprozent, insbesondere 0,1 bis 95 Gew.-%, Wirkstoff der Formel I oder Wirkstoffgemisch Antidot/Herbizid, 1 bis 99,9 Gew.-%, insbesondere 5 bis 99.8 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I oder sie enthaltender Mittel zum Schützen von Kulturpflanzen gegen schädigende Wirkungen von Herbiziden der Formel II kommen verschiedene Methoden und Techniken in Betracht, wie beispielsweise die folgenden:

i) Samenbeizung

a) Beizung der Samen mit einem als Spritzpulver formulierten Wirkstoff der Formel I durch Schütteln in einem Gefäss bis zur gleichmässigen Verteilung auf der Samenoberfläche (Trockenbeizung). Man verwendet dabei etwa 1 bis 500 g Wirkstoff der Formel I (4 g bis 2 kg Spritzpulver) pro 100 kg Saatgut.
b) Beizung der Samen mit einem Emulsionskonzentrat des Wirksoffs der Formel I nach der Methode a) (Nassbeizung).
c) Beizung durch Tauchen des Saatguts in eine Brühe mit 50-3200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 1 bis 500 g Antidot, vorzugsweise 5 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Antidot und Herbizid (gegenseitiges Mengenverhältnis zwischen 10:1 und 1:100) wird verwendet, wobei die Aufwandmenge an Herbizid 0,01 bis 1,0 kg pro Hektar beträgt. Solche Tankmischung wird vor oder nach der Aussaat appliziert.

iii) Applikation in der Saatfurche

Das Antidot wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff der Formel I wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Formulierungsbeispiele für flüssige Wirkstoffe der Formel I oder Mischungen derselben mit einem Herbizid (% = Gewichtsprozent)

| 1. Emulsions-Konzentrate | | | |
|---|---|---|---|
| | a) | b) | c) |
| Wirkstoff aus Tabelle 2 oder Mischung mit Herbizid | 25 % | 40 % | 50 % |
| Ca-Dodecylbenzolsulfonat | 5 % | 8 % | 6 % |
| Ricinusöl-polyethylenglykolether (36 Mol EO) | 5 % | - | - |
| Tributylphenol-polyethylenglykolether (30 Mol EO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | | | | |
|---|---|---|---|---|
| | a) | b) | c) | d) |
| Wirkstoff aus Tabelle 2 oder Mischung mit Herbizid | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°) | - | - | 94 % | - |

Die Lösungen sind zur Anwendung in Form kleinster Tropfen geeignet.

| 3. Granulate | | |
|---|---|---|
| | a) | b) |
| Wirkstoff aus Tabelle 2 oder Mischung mit Herbizid | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | | |
|---|---|---|
| | a) | b) |
| Wirkstoff aus Tabelle 2 oder Mischung mit Herbizid | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

| 5. Spritzpulver | a) | b) | c) |
|---|---|---|---|
| Wirkstoff aus Tabelle 2 oder Mischung mit Herbizid | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol EO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrate | |
|---|---|
| Wirkstoff aus Tabelle 2 oder Mischung mit Herbizid | 10 % |
| Octylphenolpolyethylenglykolether (4-5 Mol EO) | 3 % |
| Ca-Dodecylbenzolsulfonat | 3 % |
| Ricinusölpolyglykolether (35 Mol EO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff aus Tabelle 2 oder Mischung mit Herbizid | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 8. Extruder-Granulate | |
|---|---|
| Wirkstoff aus Tabelle 2 oder Mischung mit Herbizid | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulate | |
|---|---|
| Wirkstoff aus Tabelle 2 oder Mischung mit Herbizid | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 10. Suspensions-Konzentrate | |
|---|---|
| Wirkstoff aus Tabelle 2 oder Mischung mit Herbizid | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8% |
| Wasser | 32 % |

Der feingemahlene Wirkstoff wird mit den Zusatzstoffen innig vermischt. Man erhält so ein Suspensions-Konzentrat, aus welchen durch Verdünnen mit Wasser Suspensionen jeder gewünschten Konzentration hergestellt werden können.

Biologische Beispiele

Die Fähigkeit der Verbindungen der Formel I, Kulturpflanzen vor der phytotoxischen Wirkung starker Herbizide zu schützen, kann aus den folgenden Beispielen ersehen werden. In der Versuchsbeschreibung werden die Verbindungen der Formel I als Safener oder Gegenmittel (Antidot) bezeichnet.

Zur Bestimmung der Schutzwirkung des Safeners wird die Schädigung an der Pflanze in einem linearen neunstufigen Bonitierungsschema bewertet, wobei 1 für 100 % Schädigung und 9 für keine Schädigung (Pflanze wächst wie die unbehandelte Referenz) steht. Das Herbizid wird in einer Aufwandmenge appliziert, in welcher die Versuchspflanze teilweise geschädigt wird. Zur Bewertung der Safenerwirkung wird die Schädigung von Pflanzen, die nur mit dem Herbizid behandelt wurde und von Pflanzen, die mit dem Herbizid un dem Antidot behandelt wurden, ermittelt. Die Differenz bei der mit den Herbizid und Herbizid + Safener ermittelten Boniternoten ergibt die Schutzwirkung. Sie wird in Prozent angegeben.

Prüfung der Schutzwirkung gegen Herbizide in Getreide. Das Herbizid und das Antidot werden post-emergent als Tankmischung appliziert.

Weizensamen der Sorte "Besso" und Gerstensamen der Sorte "Cornel" werden getrennt in Plastiktöpfen (oberer Durchmesser 7 cm) in sandig tonige Lehmerde eingesät, mit Erde bedeckt und anschliessend angegossen. Die Testpflanzen werden im Gewächshaus kultiviert und im 2- bis 3-Blattstadium mit den Prüfsubstanzen behandelt.

Die als Safener zu prüfende Substanz wird in Wasser gelöst und zusammen mit dem Herbizid in 550 l Brühe/ha auf die Pflanze gesprüht.

Zwei Wochen nach der Applikation wird der Zustand der Pflanzen bonitiert. Als Referenz dienen dabei vollständig unbehandelte Kontrollen. (100 % Wachstum) Die Schadwirkung wird in Prozent evaluiert. Die Differenz des Schadens von mit dem Herbizid allein behandelten Pflanzen und solchen welche mit dem Herbizid und dem Antidot behandelt wurden wird in % Schutzwirkung ausgedrückt.

Die Resultate sind untenstehend zusammengefasst.

Das verwendete Herbizid ist 2-[4-(5-Chlor-3-fluorpyridin-2-yloxy)-phenoxy]-propionsäure-methylester, das Antidot ist mit dem der Tabelle 2 gegebenen Nummer identifiziert

| Herbizid Aufwandmenge | Antidot | | Kultur | Schutzwirkung |
|---|---|---|---|---|
| | No | Aufwandmenge | | |
| 0,5 kg/ha | 2.002 | 0,4 kg/ha | Weizen | 50 % |
| 0,5 kg/ha | 2.008 | 0,4 kg/ha | Weizen | 50 % |
| 0,5 kg/ha | 2.011 | 0,4 kg/ha | Weizen | 38 % |
| Das verwendete Herbizid ist das 2-R-Enantiomer von2-[4-(5-Chlor-3-fluorpyridin-2-yloxy)-phenoxy]-propionsäure-propinylester, | | | | |
| 0,4 kg/ha | 2.016 | 0,4 kg/ha | Weizen | 50 % |
| 0,4 kg/ha | 2.017 | 0,4 kg/ha | Weizen | 63 % |
| 0,4 kg/ha | 2.018 | 0,4 kg/ha | Weizen | 50 % |
| 0,4 kg/ha | 2.020 | 0,4 kg/ha | Weizen | 13 % |
| 0,4 kg/ha | 2.021 | 0,4 kg/ha | Weizen | 37 % |
| 0,4 kg/ha | 2.022 | 0,4 kg/ha | Weizen | 50 % |

In einer ausgedehnten Versuchsanordnung, bei der man den Zustand der Pflanzen erst nach 3 Wochen auswertete, wurden folgende Resultate ermittelt:
Kultur: Sommerweizen "Besso"
Herbizid: 2-[4-(5-Chlor-3-fluorpyridin-2-yloxy)-phenoxy]-propionsäuremethylester

| Herbizid Aufwandmenge | Antidot | | Schutzwirkung |
|---|---|---|---|
| | No | Aufwandmenge | |
| 0.5 kg/ha | - | | 0 % |
| 0.5 kg/ha | 2.002 | 0.5 kg/ha | 63 % |
| 0.5 kg/ha | 2.002 | 0.25 kg/ha | 63 % |
| 0.5 kg/ha | 2.002 | 0.125 kg/ha | 63 % |
| 0.5 kg/ha | 2.002 | 0.062 kg/ha | 50 % |
| 0.25 kg/ha | 2.002 | 0.25 kg/ha | 63 % |
| 0.25 kg/ha | 2.002 | 0.125 kg/ha | 75 % |
| 0.25 kg/ha | 2.002 | 0.062 kg/ha | 63 % |
| 0.25 kg/ha | 2.002 | 0.031 kg/ha | 75 % |
| 0.125 kg/ha | 2.002 | 0.125 kg/ha | 50 % |
| 0.125 kg/ha | 2.002 | 0.062 kg/ha | 38 % |
| 0.125 kg/ha | 2.002 | 0.031 kg/ha | 63 % |
| 0.125 kg/ha | 2.002 | 0.016 kg/ha | 38 % |
| 0.5 kg/ha | 2.010 | 0.25 kg/ha | 50 % |
| 0.5 kg/ha | 2.010 | 0.125 kg/ha | 63 % |
| 0.5 kg/ha | 2.010 | 0.062 kg/ha | 63 % |
| 0.25 kg/ha | 2.010 | 0.25 kg/ha | 63 % |
| 0.25 kg/ha | 2.010 | 0.125 kg/ha | 63 % |
| 0.25 kg/ha | 2.010 | 0.062 kg/ha | 63 % |
| 0.25 kg/ha | 2.010 | 0.031 kg/ha | 50 % |
| 0.125 kg/ha | 2.010 | 0.125 kg/ha | 38 % |
| 0.125 kg/ha | 2.010 | 0.062 kg/ha | 50 % |
| 0.125 kg/ha | 2.010 | 0.031 kg/ha | 50 % |
| 0.125 kg/ha | 2.010 | 0.016 kg/ha | 50 % |

Kultur: Sommerweizen "Besso"
Herbizid: 2-[4-(5-Chlor-3-fluorpyridin-2-yloxy)-phenoxy]-propionsäurepropinylester

| Herbizid Aufwandmenge | Antidot | | Schutzwirkung |
|---|---|---|---|
| | No | Aufwandmenge | |
| 0.5 kg/ha | 2.002 | 0.5 kg/ha | 63 % |
| 0.5 kg/ha | 2.002 | 0.25 kg/ha | 63 % |
| 0.5 kg/ha | 2.002 | 0.125 kg/ha | 63 % |
| 0.5 kg/ha | 2.002 | 0.062 kg/ha | 75 % |
| 0.25 kg/ha | 2.002 | 0.25 kg/ha | 63 % |
| 0.25 kg/ha | 2.002 | 0.125 kg/ha | 63 % |
| 0.25 kg/ha | 2.002 | 0.062 kg/ha | 50 % |
| 0.25 kg/ha | 2.002 | 0.031 kg/ha | 63 % |
| 0.5 kg/ha | 2.010 | 0.25 kg/ha | 25 % |
| 0.5 kg/ha | 2.010 | 0.125 kg/ha | 38 % |
| 0.5 kg/ha | 2.010 | 0.062 kg/ha | 13 % |
| 0.25 kg/ha | 2.010 | 0.25 kg/ha | 63 % |
| 0.25 kg/ha | 2.010 | 0.125 kg/ha | 75 % |
| 0.25 kg/ha | 2.010 | 0.062 kg/ha | 75 % |
| 0.25 kg/ha | 2.010 | 0.031 kg/ha | 75 % |
| 0.125 kg/ha | 2.010 | 0.125 kg/ha | 25 % |
| 0.125 kg/ha | 2.010 | 0.062 kg/ha | 25 % |
| 0.125 kg/ha | 2.010 | 0.031 kg/ha | 38 % |
| 0.125 kg/ha | 2.010 | 0.016 kg/ha | 38 % |

Kultur: Sommergerste "Cornel"
Herbizid: 2-[4-(5-Chlor-3-fluorpyridin-2-yloxy)-phenoxy]-propionsäuremethylester

| Herbizid Aufwandmenge | Antidot | | Schutzwirkung |
|---|---|---|---|
| | No | Aufwandmenge | |
| 0.25 kg/ha | 2.002 | 0.25 kg/ha | 50 % |
| 0.25 kg/ha | 2.002 | 0.125 kg/ha | 38 % |
| 0.25 kg/ha | 2.002 | 0.062 kg/ha | 25 % |
| 0.25 kg/ha | 2.002 | 0.031 kg/ha | 38 % |
| 0.125 kg/ha | 2.002 | 0.125 kg/ha | 38 % |
| 0.125 kg/ha | 2.002 | 0.062 kg/ha | 50 % |
| 0.125 kg/ha | 2.002 | 0.031 kg/ha | 50 % |
| 0.125 kg/ha | 2.002 | 0.016 kg/ha | 38 % |

Kultur: Sommergerste "Cornel"
Herbizid: 2-[4-(5-Chlor-3-fluorpyridin-2-yloxy)-phenoxy]-propionsäurepropinylester

| Herbizid Aufwandmenge | Antidot | | Schutzwirkung |
|---|---|---|---|
| | No | Aufwandmenge | |
| 0.5 kg/ha | 2.002 | 0.5 kg/ha | 38 % |
| 0.5 kg/ha | 2.002 | 0.25 kg/ha | 38 % |
| 0.5 kg/ha | 2.002 | 0.125 kg/ha | 50 % |
| 0.5 kg/ha | 2.002 | 0.062 kg/ha | 63 % |
| 0.25 kg/ha | 2.002 | 0.25 kg/ha | 75 % |
| 0.25 kg/ha | 2.002 | 0.125 kg/ha | 75 % |
| 0.25 kg/ha | 2.002 | 0.062 kg/ha | 63 % |
| 0.25 kg/ha | 2.002 | 0.031 kg/ha | 75 % |
| 0.125 kg/ha | 2.002 | 0.125 kg/ha | 50 % |
| 0.125 kg/ha | 2.002 | 0.062 kg/ha | 50 % |
| 0.125 kg/ha | 2.002 | 0.031 kg/ha | 50 % |
| 0.125 kg/ha | 2.002 | 0.016 kg/ha | 50 % |
| 0.125 kg/ha | 2.010 | 0.125 kg/ha | 38 % |
| 0.125 kg/ha | 2.010 | 0.062 kg/ha | 38 % |
| 0.125 kg/ha | 2.010 | 0.031 kg/ha | 13 % |
| 0.125 kg/ha | 2.010 | 0.016 kg/ha | 13 % |

Kultur: Sommergerste "Cornel"
Herbizid: N-Benzoyl-N-(3-chlor-4-fluorphenyl)alanin-methylester

| Herbizid Aufwandmenge | Antidot | | Schutzwirkung |
|---|---|---|---|
| | No | Aufwandmenge | |
| 1 kg/ha | 2.002 | 0.2 kg/ha | 55 % |
| 1 kg/ha | 2.002 | 0.1 kg/ha | 60 % |
| 0.5 kg/ha | 2.002 | 0.2 kg/ha | 30 % |
| 0.5 kg/ha | 2.002 | 0.1 kg/ha | 35 % |
| 1 kg/ha | 2.022 | 0.2 kg/ha | 35 % |
| 1 kg/ha | 2.022 | 0.1 kg/ha | 35 % |
| 0.5 kg/ha | 2.022 | 0.2 kg/ha | 15 % |
| 0.5 kg/ha | 2.022 | 0.1 kg/ha | 20 % |
| 1 kg/ha | 2.034 | 0.2 kg/ha | 40 % |
| 1 kg/ha | 2.034 | 0.1 kg/ha | 50 % |
| 0.5 kg/ha | 2.034 | 0.2 kg/ha | 30 % |
| 0.5 kg/ha | 2.034 | 0.1 kg/ha | 25 % |

Kultur: Sommerweizen "Besso"
Herbizid: Das 2R Enantiomer des 2-[4-(5-Chlor-3-fluorpyridin-2-yloxy)-phenoxy]-propionsäure-propinylesters

| Herbizid Aufwandmenge | Antidot | | Schutzwirkung |
|---|---|---|---|
| | No | Aufwandmenge | |
| 400 g/ha | 2.016 | 400 g/ha | 75 % |
| 400 g/ha | 2.016 | 200 g/ha | 63 % |
| 400 g/ha | 2.016 | 100 g/ha | 50 % |
| 400 g/ha | 2.016 | 50 g/ha | 38 % |
| 200 g/ha | 2.016 | 200 g/ha | 63 % |
| 200 g/ha | 2.016 | 100 g/ha | 63 % |
| 200 g/ha | 2.016 | 50 g/ha | 63 % |
| 200 g/ha | 2.016 | 25 g/ha | 38 % |
| 100 g/ha | 2.016 | 100 g/ha | 38 % |
| 100 g/ha | 2.016 | 50 g/ha | 38 % |
| 100 g/ha | 2.016 | 25 g/ha | 38 % |
| 100 g/ha | 2.016 | 13 g/ha | 38 % |
| 400 g/ha | 2.017 | 400 g/ha | 63 % |
| 400 g/ha | 2.017 | 200 g/ha | 75 % |
| 400 g/ha | 2.017 | 100 g/ha | 50 % |
| 400 g/ha | 2.017 | 50 g/ha | 38 % |
| 200 g/ha | 2.017 | 200 g/ha | 63 % |
| 200 g/ha | 2.017 | 100 g/ha | 75 % |
| 200 g/ha | 2.017 | 50 g/ha | 63 % |
| 200 g/ha | 2.017 | 25 g/ha | 50 % |
| 100 g/ha | 2.017 | 100 g/ha | 50 % |
| 100 g/ha | 2.017 | 50 g/ha | 50 % |
| 100 g/ha | 2.017 | 25 g/ha | 50 % |
| 100 g/ha | 2.017 | 13 g/ha | 38 % |

Kultur: Sommerweizen "Besso"
Herbizid: Das 2R Enantiomer des 2-[4-(5-Chlor-3-fluorpyridin-2-yloxy)-phenoxy]-propionsäure-propinylesters

| Herbizid Aufwandmenge | Antidot | | Schutzwirkung |
|---|---|---|---|
| | No | Aufwandmenge | |
| 400 g/ha | 2.018 | 400 g/ha | 75 % |
| 400 g/ha | 2.018 | 200 g/ha | 75 % |
| 400 g/ha | 2.018 | 100 g/ha | 63 % |
| 400 g/ha | 2.018 | 50 g/ha | 63 % |
| 200 g/ha | 2.018 | 200 g/ha | 63 % |
| 200 g/ha | 2.018 | 100 g/ha | 63 % |
| 200 g/ha | 2.018 | 50 g/ha | 75 % |
| 200 g/ha | 2.018 | 25 g/ha | 75 % |
| 100 g/ha | 2.018 | 100 g/ha | 38 % |
| 100 g/ha | 2.018 | 50 g/ha | 50 % |
| 100 g/ha | 2.018 | 25 g/ha | 63 % |
| 100 g/ha | 2.018 | 13 g/ha | 63 % |
| 400 g/ha | 2.022 | 400 g/ha | 50 % |
| 400 g/ha | 2.022 | 200 g/ha | 50 % |
| 400 g/ha | 2.022 | 100 g/ha | 38 % |
| 400 g/ha | 2.022 | 50 g/ha | 25 % |
| 200 g/ha | 2.022 | 200 g/ha | 63 % |
| 200 g/ha | 2.022 | 100 g/ha | 63 % |
| 200 g/ha | 2.022 | 50 g/ha | 63 % |
| 200 g/ha | 2.022 | 25 g/ha | 63 % |
| 100 g/ha | 2.022 | 100 g/ha | 50 % |
| 100 g/ha | 2.022 | 50 g/ha | 63 % |
| 100 g/ha | 2.022 | 25 g/ha | 63 % |
| 100 g/ha | 2.022 | 13 g/ha | 63 % |

Kultur: Sommerweizen "Besso"
Herbizid: Das 2R Enantiomer des 2-[4-(5-Chlor-3-fluorpyridin-2-yloxy)-phenoxy]-propionsäure-propinylesters

| Herbizid Aufwandmenge | Antidot | | Schutzwirkung |
|---|---|---|---|
| | No | Aufwandmenge | |
| 200 g/ha | A | 200 g/ha | 25 % |
| 200 g/ha | A | 100 g/ha | 25 % |
| 200 g/ha | A | 25 g/ha | 25 % |
| 100 g/ha | A | 100 g/ha | 38 % |
| 100 g/ha | A | 50 g/ha | 25 % |
| 100 g/ha | A | 25 g/ha | 38 % |
| 100 g/ha | A | 13 g/ha | 38 % |
| Antidot A ist 1-(3-Chlorphenyl)-5-trichlormethyl-1,2,4-triazol-3-carbonsäureethylester, bekannt aus der DE-A 3.525.505. | | | |

Kultur: Sommergerste "Cornel"
Herbizid: Das 2R Enantiomer des 2-[4-(5-Chlor-3-fluorpyridin-2-yloxy)-phenoxy]-propionsäure-propinylesters

| Herbizid Aufwandmenge | Antidot | | Schutzwirkung |
|---|---|---|---|
| | No | Aufwandmenge | |
| 400 g/ha | 2.016 | 400 g/ha | 63 % |
| 400 g/ha | 2.016 | 200 g/ha | 50 % |
| 400 g/ha | 2.016 | 100 g/ha | 38 % |
| 400 g/ha | 2.016 | 50 g/ha | 13 % |
| 200 g/ha | 2.016 | 200 g/ha | 38 % |
| 200 g/ha | 2.016 | 100 g/ha | 50 % |
| 200 g/ha | 2.016 | 50 g/ha | 25 % |
| 100 g/ha | 2.016 | 100 g/ha | 50 % |
| 100 g/ha | 2.016 | 50 g/ha | 50 % |
| 100 g/ha | 2.016 | 25 g/ha | 50 % |
| 100 g/ha | 2.016 | 13 g/ha | 38 % |
| 400 g/ha | 2.017 | 400 g/ha | 50 % |
| 400 g/ha | 2.017 | 200 g/ha | 38 % |
| 200 g/ha | 2.017 | 200 g/ha | 63 % |
| 200 g/ha | 2.017 | 100 g/ha | 13 % |
| 100 g/ha | 2.017 | 100 g/ha | 50 % |
| 100 g/ha | 2.017 | 50 g/ha | 13 % |

Kultur: Sommergerste "Cornel"
Herbizid: Das 2R Enantiomer des 2-[4-(5-Chlor-3-fluorpyridin-2-yloxy)-phenoxy]-propionsäure-propinylesters

| Herbizid Aufwandmenge | Antidot | | Schutzwirkung |
|---|---|---|---|
| | No | Aufwandmenge | |
| 400 g/ha | 0018 | 400 g/ha | 50 % |
| 400 g/ha | 0018 | 200 g/ha | 25 % |
| 200 g/ha | 0018 | 200 g/ha | 38 % |
| 200 g/ha | 0018 | 100 g/ha | 13 % |
| 100 g/ha | 0018 | 100 g/ha | 38 % |
| 100 g/ha | 0018 | 50 g/ha | 13 % |
| 100 g/ha | 0018 | 25 g/ha | 25 % |

Kultur: Sommergerste "Cornel"
Herbizid: 2R-2-[4-(5-Chlor-3-fluorpyridin-2-yloxy)-phenoxy]-propionsäure-propinylester

| Herbizid Aufwandmenge | Antidot | | Schutzwirkung |
|---|---|---|---|
| | No | Aufwandmenge | |
| 400 g/ha | A | 400 g/ha | 0 % |
| 400 g/ha | A | 200 g/ha | 13 % |
| 400 g/ha | A | 100 g/ha | 0 % |
| 400 g/ha | A | 50 g/ha | 0 % |
| 200 g/ha | A | 200 g/ha | 0 % |
| 200 g/ha | A | 100 g/ha | 13 % |
| 200 g/ha | A | 50 g/ha | 0 % |
| 200 g/ha | A | 25 g/ha | 0 % |
| 100 g/ha | A | 100 g/ha | 25 % |
| 100 g/ha | A | 50 g/ha | 13 % |
| 100 g/ha | A | 25 g/ha | 25 % |
| 100 g/ha | A | 13 g/ha | 25 % |
| Antidot A ist 1-(3-Chlorphenyl)-5-trichlormethyl-1,2,4-triazol-3-carbonsäureethylester, bekannt aus der DE-A 3.505.205. | | | |

2. Prüfung der Schutzwirkung gegen Herbizide in Hirse.

2.1 Das Antidot wird durch Saatbeizung, das Herbizid im Vorauflaufverfahren appliziert.

Sorghumsamen werden mit der als Antidot zu prüfenden Substanz in einen Glasbehälter gemischt. Samen und Produkt werden durch Schütteln und Rotation gut zusammengemischt. Töpfe desselben Formats (oberer ∅ 11 cm) werden mit Erde gefüllt und die gebeizten Samen werden eingesät. Nach dem Bedecken der Samen wird das Herbizid im Vorauflauf appliziert. 14 Tage nach der Herbizidapplikation wird die Schutzwirkung des Antidot in Prozent bonitiert. Als Referenzen dienen dabei vollständig unbehandelte Kontrollpflanzen.

Die Resultate sind wie folgt:
Kultur: Sorghum der Sorte Funk G-623
Herbizid: N-Chloracetyl-N-(2-methoxy-1-methylethyl)-2-ethyl-6-methylanilin

| Herbizid Aufwandmenge | Antidot | | Schutzwirkung |
|---|---|---|---|
| | No | Aufwandmenge | |
| 4 kg/ha | 2.002 | 2 g/kg | 50 % |
| 4 kg/ha | 2.002 | 0.5 g/kg | 13 % |
| 4 kg/ha | 2.002 | 0.125 g/kg | 13 % |
| 4 kg/ha | 2.002 | 0.031 g/kg | 13 % |
| 2 kg/ha | 2.002 | 2 g/kg | 63 % |
| 2 kg/ha | 2.002 | 0.5 g/kg | 50 % |
| 2 kg/ha | 2.002 | 0.125 g/kg | 25 % |
| 2 kg/ha | 2.002 | 0.031 g/kg | 13 % |
| 1 kg/ha | 2.002 | 2 g/kg | 63 % |
| 1 kg/ha | 2.002 | 0.5 g/kg | 63 % |
| 1 kg/ha | 2.002 | 0.125 g/kg | 50 % |
| 1 kg/ha | 2.002 | 0.031 g/kg | 38 % |

2.2 Das Herbizid und das Antidot werden pre-emergent als Tankmischung appliziert.

Hirsesamen der Sorte Funk G-623 werden in Plastiktöpfen (oberer Durchmesser 7 cm) in sandig tonige Lehmerde eingesät, mit Erde bedeckt und anschliessend angegossen.

Die als Safener zu prüfende Substanz wird in Wasser gelöst und zusammen mit dem Herbizid in 550 l

Brühe/ha pre-emergent auf den Boden gesprüht.

Zwei Wochen nach der Applikation wird die Schutzwirkung des Safeners bonitiert. Als Referenz dienen dabei vollständig unbehandelte Kontrollpflanzen. (100 % Wachstum) Die Schadwirkung wird in Prozent evaluiert. Die Differenz des Schadens von mit dem Herbizid allein behandelten Pflanzen und solchen welche mit dem Herbizid und dem Antidot behandelt wurden wird in % Schutzwirkung ausgedrückt.

Die Resultate sind untenstehend zusammengefasst.

Das verwendete Herbizid für Hirse ist N-chloracetyl-N-(2-methoxy-1-methylethyl)2-ethyl-6-chloranilin. Das Antidot ist durch die in der Tabelle 2 gegebene Nummer identifiziert

| Herbizid Aufwandmenge | Antidot | | Kultur Schutzwirkung |
|---|---|---|---|
| | No | Aufwandmenge | |
| 1,5 kg/ha | 2.002 | 1,5 kg/ha | 38 % |
| 1,5 kg/ha | 2.016 | 1,5 kg/ha | 25 % |
| 1,5 kg/ha | 2.020 | 1,5 kg/ha | 25 % |
| 1,5 kg/ha | 2.022 | 1,5 kg/ha | 13 % |

In einer ausgedehnten Versuchsanordnung bei der man den Zustand der Pflanzen erst nach 3 Wochen auswertete, wurden folgende Resultate ermittelt:

| Herbizid Aufwandmenge | Antidot | | Schutzwirkung |
|---|---|---|---|
| | No | Aufwandmenge | |
| 4 kg/ha | 2.002 | 4 kg/ha | 25 % |
| 4 kg/ha | 2.002 | 2 kg/ha | 13 % |
| 4 kg/ha | 2.002 | 1 kg/ha | 13 % |
| 2 kg/ha | 2.002 | 2 kg/ha | 13 % |
| 2 kg/ha | 2.002 | 1 kg/ha | 13 % |
| 1 kg/ha | 2.002 | 1 kg/ha | 38 % |
| 1 kg/ha | 2.002 | 0,5 kg/ha | 25 % |

## Patentansprüche

1. Verfahren zum Schützen von Kulturpflanzen vor der phytotoxischen Wirkung von Herbiziden, dadurch gekennzeichnet, dass man die Pflanzen, deren Anbaufläche, oder deren Saatgut zusammen mit dem Herbizid oder unabhängig davon mit einer herbizid-antagonistisch wirksamen Menge eines 1,5-Diphenyl-1,2,4-triazol-3-carbonsäurederivat der Formel I

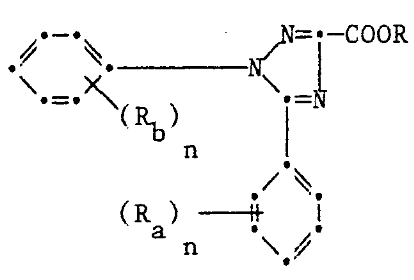

(I)

worin $R_a$ und $R_b$ unabhängig voneinander je Halogen, $C_1$-$C_5$-Alkyl, $C_1$-$C_5$-Halogenalkyl, $C_2$-$C_5$-Alkenyl, $C_2$-$C_5$-Alkinyl, $C_1$-$C_5$-Alkoxy, $C_1$-$C_5$-Halogenalkoxy, Nitro oder Cyano,

n Null oder eine Zahl von 1 bis 3 und

R Wasserstoff, ein pflanzenphysiologisch verträgliches Metall- oder Ammoniumkation, $C_1$-$C_8$-Alkyl oder $C_3$-$C_{12}$-Cycloalkyl welches unsubstituiert oder ein- oder mehrfach durch $C_1$-$C_4$-Alkoxy, $C_1$-$C_4$-Alkylthio, Mono- oder Di-$C_1$-$C_4$-alkylamino, Amino oder $C_1$-$C_4$-Alkoxycarbonyl substituiert ist; $C_3$-$C_6$-Alkenyl oder $C_3$-$C_{12}$-Cycloalkenyl, welches unsubstituiert oder ein-oder mehrfach durch Halogen substituiert ist,

$C_3$-$C_6$-Alkinyl; Phenyl oder Benzyl, welches unsubstituiert oder ein-oder mehrfach durch Halogen, Nitro, $C_1$-$C_4$-Alkyl, $C_1$-$C_4$-Alkoxy, $C_1$-$C_4$-Alkylthio, $C_1$-$C_4$-Haloalkyl, $C_1$-$C_4$-Haloalkoxy oder Cyano substituiert ist, bedeuten, behandelt.

2. Mittel zur selektiven Kontrolle von Unkräutern und Gräsern in Nutzpflanzen-Kulturen, dadurch gekennzeichnet, dass es neben inerten Trägerstoffen und Zusatzmaterialien als Wirkstoff ein Herbizid und eine herbizid-antagonistisch wirksame Menge eines 1,5-Diphenyl-1,2,4-triazol-3-carbonsäurederivates der Formel I gemäss Anspruch 1 enthält.

3. Verfahren zum selektiven Bekämpfen von Unkräutern und Gräsern in Nutzpflanzenkulturen, dadurch gekennzeichnet, dass man die Kulturen oder deren Anbaufläche gleichzeitig oder unabhängig voneinander mit einer wirksamen Menge eines Herbizides und einer herbizid-antagonistisch wirksamen Menge eines 1,5-Diphenyl-1,2,4-triazol-3-carbonsäurederivates der Formel I gemäss Anspruch 1 behandelt.

4. 1,5-Diphenyl-1,2,4-triazol-3-carbonsäurederivate der Formel I, Anspruch 1, mit der Massgabe, dass keiner der Substituenten $(R_a)_n$ und $(R_b)_n$ Nitro und mindestens einer ein Chlor oder Fluoratom bedeutet.

5. 1,5-Diphenyl-1,2,4-triazol-3-carbonsäurederivate der Formel Ia

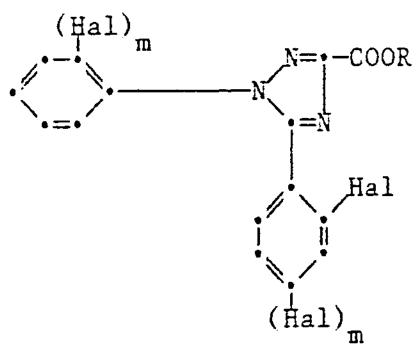

(Ia)

worin Hal ein Halogenatom,
m Null oder 1 bedeuten und
R die im Anspruch 1 gegebene Bedeutung hat.

6. Verfahren zur Herstellung von 1,5-Diphenyl-1,2,4-triazol-3-carbonsäurederivaten der Formel Ia

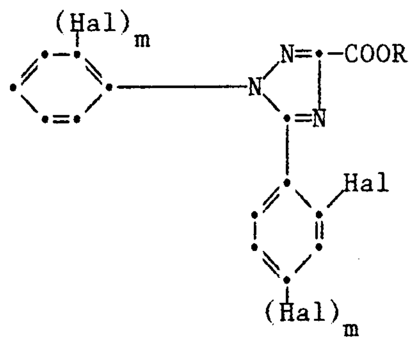

(Ia)

worin Hal ein Halogenatom,
m Null oder 1 bedeuten und
R die im Anspruch 1 gegebene Bedeutung hat, dadurch gekennzeichnet, dass man ein Anilin der Formel

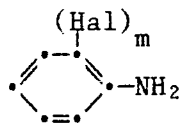

worin Hal und m die oben gegebene Bedeutung haben, mit Natriumnitrit und Salzsäure diazotiert und zum erhaltenen Diazoniumsalz einen Benzoylaminomalonsäuredialkylester der Formel VI

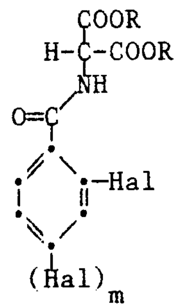

(VI)

worin Hal und m die oben gegebene, R die im Anspruch 1 gegebene Bedeutung hat, zufügt und das Kondensationsprodukt unter wasserabspaltenden Bedingungen ringschliesst.

**7.** Mittel zum Schützen von Kulturpflanzen vor der phytotoxischen Wirkung von Herbiziden, dadurch gekennzeichnet, dass es neben inerten Trägerstoffen und Zusatzmaterialien als herbizid-antagonistischen Wirkstoff ein 1,5-Diphenyl-1,2,4-triazol-3-carbonsäurederivat der Formel I gemäss Anspruch 5 enthält.

**8.** 1,5-Diphenyl-1,2,4-triazol-3-carbonsäurederivate der Formel Ib

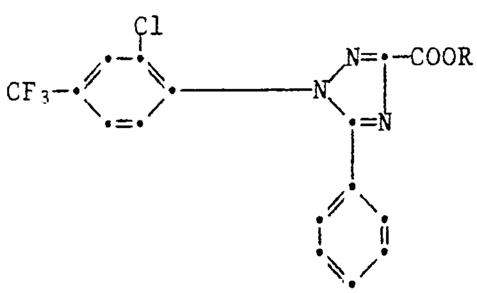

(Ib)

worin R die im Anspruch 1 gegebene Bedeutung hat.

**9.** 1-(2-Chlor-4-trifluormethylphenyl)-5-phenyl-1,2,4-triazol-3-carbonsäure-methylester gemäss Anspruch 6.

**10.** 1-(2-Chlor-4-trifluormethylphenyl)-5-phenyl-1,2,4-triazol-3-carbonsäure-ethylester gemäss Anspruch 6.

## Claims

**1.** A method of protecting cultivated plants from the phytotoxic action of herbicides, which comprises treating the plants, the cultivation area thereof or the seed thereof with a herbicide-safening amount of a 1,5-diphenyl-1,2,4-triazole-3-carboxylic acid derivative of formula I

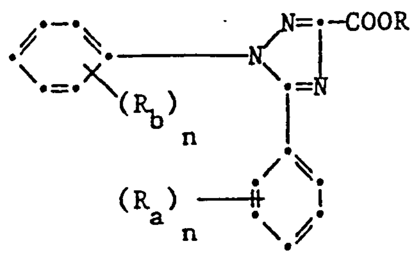

(I)

wherein

each of $R_a$ and $R_b$, independently of the other, is halogen, $C_1$-$C_5$alkyl, $C_1$-$C_5$haloalkyl, $C_2$-$C_5$-alkenyl, $C_2$-$C_5$alkynyl, $C_1$-$C_5$alkoxy, $C_1$-$C_5$haloalkoxy, nitro or cyano,

n is zero or a number from 1 to 3 and

R is hydrogen; a phytophysiologically tolerable metal or ammonium cation; $C_1$-$C_8$alkyl or $C_3$-$C_{12}$cycloalkyl each of which is unsubstituted or is mono- or poly-substituted by $C_1$-$C_4$alkoxy, $C_1$-$C_4$alkylthio, mono- or di-$C_1$-$C_4$-alkylamino, amino or by $C_1$-$C_4$alkoxycarbonyl; $C_3$-$C_6$alkenyl or $C_3$-$C_{12}$cycloalkenyl each of which is unsubstituted or is mono- or poly-substituted by halogen; $C_3$-$C_6$alkynyl; or phenyl or benzyl each of which is unsubstituted or is mono- or poly-substituted by halogen, nitro, $C_1$-$C_4$alkyl, $C_1$-$C_4$alkoxy, $C_1$-$C_4$alkylthio, $C_1$-$C_4$haloalkyl, $C_1$-$C_4$haloalkoxy or by cyano,

together with the herbicide or independently thereof.

2. A composition for the selective control of weeds and grasses in crops of useful plants, which comprises, as active ingredient, a herbicide and a herbicide-safening amount of a 1,5-diphenyl-1,2,4-triazole-3-carboxylic acid derivative of formula I according to claim 1, together with inert carriers and adjuvants.

3. A method of selectively controlling weeds and grasses in crops of useful plants, which comprises treating the crops or the cultivation area thereof with an effective amount of a herbicide and a herbicide-safening amount of a 1,5-diphenyl-1,2,4-triazole-3-carboxylic acid derivative of formula I according to claim 1, simultaneously with or independently of each other.

4. A 1,5-diphenyl-1,2,4-triazole-3-carboxylic acid derivative of formula I, claim 1, with the proviso that none of the substituents $(R_a)_n$ and $(R_b)_n$ is nitro and at least one is a chlorine or fluorine atom.

5. A 1,5-diphenyl-1,2,4-triazole-3-carboxylic acid derivative of formula Ia

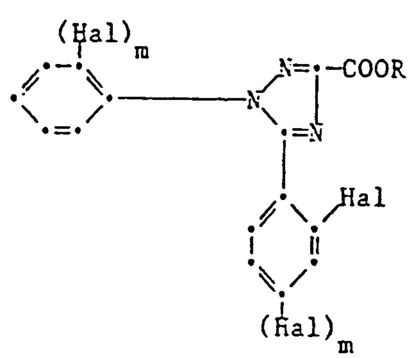

(Ia)

wherein

Hal is a halogen atom,

m is zero or 1 and

R is as defined in claim 1.

6. A process for the preparation of a 1,5-diphenyl-1,2,4-triazole-3-carboxylic acid derivative of formula Ia

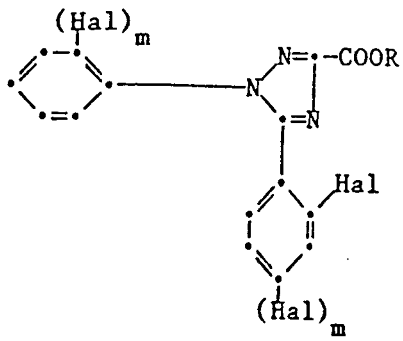

(Ia)

wherein

Hal is a halogen atom,
m is zero or 1, and
R is as defined in claim 1,

which process comprises diazotising an aniline of the formula

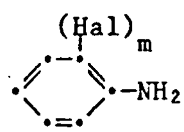

wherein Hal and m are as defined above, with sodium nitrite and hydrochloric acid and adding to the resulting diazonium salt a benzoylaminomalonic acid dialkyl ester of formula VI

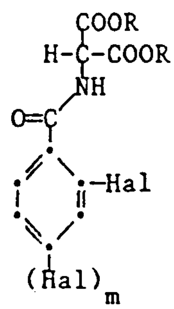

(VI)

wherein Hal and m are as defined above and R is as defined in claim 1, and cyclising the condensation product under conditions that split off the elements of water.

7. A composition for protecting cultivated plants from the phytotoxic action of herbicides, which comprises, as herbicide-safening active ingredient, a 1,5-diphenyl-1,2,4-triazole-3-carboxylic acid derivative of formula I according to claim 5, together with inert carriers and adjuvants.

8. A 1,5-diphenyl-1,2,4-triazole-3-carboxylic acid derivative of formula Ib

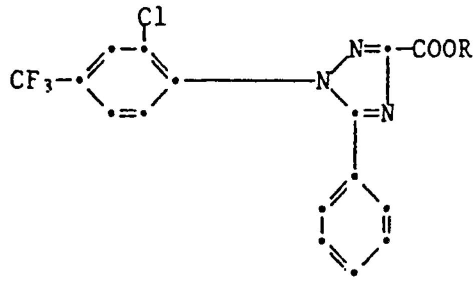

(Ib)

wherein R is as defined in claim 1.

**9.** 1-(2-Chloro-4-trifluoromethylphenyl)-5-phenyl-1,2,4-triazole-3-carboxylic acid methyl ester according to claim 6.

**10.** 1-(2-Chloro-4-trifluoromethylphenyl)-5-phenyl-1,2,4-triazole-3-carboxylic acid ethyl ester according to claim 6.

## Revendications

**1.** Procédé pour la protection des plantes cultivées contre l'action phytotoxique des herbicides, caractérisé en ce que l'on traite les plantes, les surfaces cultivées avec ces plantes ou leurs semences, ensemble avec l'herbicide, ou, indépendamment de l'herbicide, avec une quantité efficace comme antagoniste de l'herbicide d'un dérivé de l'acide 1,5-diphényl-1,2,4-triazol-3-carboxylique de formule I

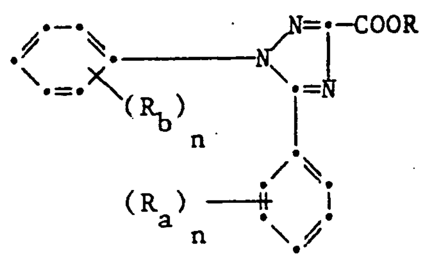

(I)

ou $R_a$ et $R_b$ représentent, indépendamment l'un de l'autre, un halogène, un alkyle en $C_1$-$C_5$, un halogénoalkyle en $C_1$-$C_5$, un alcényle en $C_2$-$C_5$, un alcynyle en $C_2$-$C_5$, un alcoxy en $C_1$-$C_5$, un halogénoalcoxy en $C_1$-$C_5$, un nitro ou un cyano,
n est égal à 0 ou est un nombre allant de 1 à 3 et
R représente l'hydrogène, un cation métallique ou un cation ammonium physiologiquement compatible avec les plantes, un alkyle en $C_1$-$C_8$ ou un cycloalkyle en $C_3$-$C_{12}$ non substitué ou une ou plusieurs fois substitué par un alcoxy en $C_1$-$C_4$, un alkyle en $C_1$-$C_4$ thio, un mono- ou dialkyle en $C_1$-$C_4$ amino, un amino ou un alcoxy en $C_1$-$C_4$ carbonyle ; un alcényle en $C_3$-$C_6$ ou un cycloalcényle en $C_3$-$C_{12}$ non substitué ou une ou plusieurs fois substitué par un halogène, un alcynyle en $C_3$-$C_6$ ; le phényle ou le benzyle non substitué ou une ou plusieurs fois substitué par un halogène, le nitro, un alkyle en $C_1$-$C_4$, un alcoxy en $C_1$-$C_4$, un alkyle en $C_1$-$C_4$ thio, un haloalkyle en $C_1$-$C_4$, un haloalcoxy en $C_1$-$C_4$ ou un cyano.

**2.** Agent pour le contrôle sélectif des mauvaises herbes et graminées dans les cultures de plantes utiles, caractérisé en ce qu'il contient, à titre de substances actives, à côté de supports inertes et d'additifs, un herbicide et une quantité efficace comme antagoniste de l'herbicide d'un dérivé de l'acide 1,5-diphényl-1,2,4-triazol-3-carboxylique de formule I selon la revendication 1.

**3.** Procédé pour combattre sélectivement les mauvaises herbes et graminées dans les cultures de plantes utiles, caractérisé en ce que l'on traite les cultures ou les surfaces cultivées, en même temps, ou, de façon indépendante, avec une quantité efficace d'un herbicide et avec une quantité efficace comme antagoniste de l'herbicide d'un dérivé de l'acide 1,5-diphényl-1,2,4-triazol-3-carboxylique de formule I

selon la revendication 1.

4. Les dérivés de l'acide 1,5-diphényl-1,2,4-triazol-3-carboxylique de formule I selon la revendication 1, sous réserve qu'aucun des substituants $(R_a)_n$ et $(R_b)_n$ représente le nitro et sous réserve qu'au moins l'un des substituants représente un atome de chlore ou de fluor.

5. Les dérivés de l'acide 1,5-diphényl-1,2,4-triazol-3-carboxylique de formule Ia

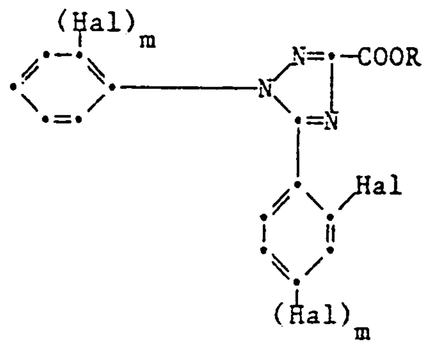

(Ia)

où Hal représente un atome d'halogène,
n égal 0 ou 1 et
R a la signification donnée dans la revendication 1.

6. Procédé pour la préparation des dérivés de l'acide 1,5-diphényl-1,2,4-triazol-3-carboxylique de formule Ia

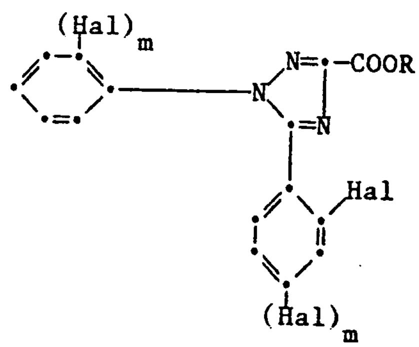

(Ia)

où Hal représente un atome d'halogène,
n égal 0 ou 1 et
R a la signification donnée dans la revendication 1, caractérisé en ce que l'on diazote une aniline de formule

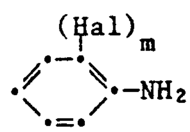

où Hal et m ont la signification donnée ci-dessus, avec du nitrite de sodium et de l'acide chlorhydrique et en ce que l'on additionne au sel de diazonium obtenu un ester dialkylique de l'acide benzoylamino-malonique de formule VI

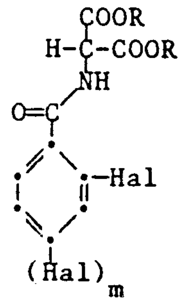

(VI)

où Hal et m ont la signification donnée ci-dessus, R a la signification donnée dans la revendication 1 et en ce que l'on cyclise le produit de condensation dans des conditions pour lesquelles il se produit une élimination d'eau.

**7.** Agent pour la protection des plantes cultivées contre l'action phytotoxique des herbicides, caractérisé en ce qu'il contient, à côté de supports inertes et d'additifs, à titre de substance active antagoniste de l'herbicide, un dérivé de l'acide 1,5-diphényl-1,2,4-triazol-3-carboxylique de formule I selon la revendication 5.

**8.** Les dérivés de l'acide 1,5-diphényl-1,2,4-triazol-3-carboxylique de formule Ib

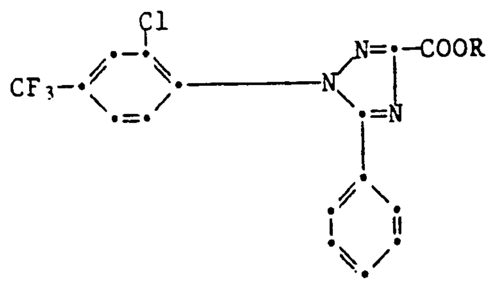

(Ib)

où R a la signification donnée dans la revendication 1.

**9.** L'ester méthylique de l'acide 1-(2-chloro-4-trifluorométhylphényl)-5-phényl-1,2,4-triazol-3-carboxylique selon la revendication 6.

**10.** L'ester éthylique de l'acide 1-(2-chloro-4-trifluorométhylphényl)-5-phényl-1,2,4-triazol-3-carboxylique selon la revendication 6.